(19) Europäisches Patentamt
European Patent Office
Office européen des brevets

(11) **EP 3 908 282 B1**

(12) **EUROPEAN PATENT SPECIFICATION**

(45) Date of publication and mention of the grant of the patent:
**02.07.2025 Bulletin 2025/27**

(21) Application number: **20703606.2**

(22) Date of filing: **07.01.2020**

(51) International Patent Classification (IPC):
***A61K 31/506*** *(2006.01)* ***A61K 45/06*** *(2006.01)*
***A61P 35/00*** *(2006.01)*

(52) Cooperative Patent Classification (CPC):
**A61K 31/506; A61K 45/06; A61P 35/00**

(86) International application number:
**PCT/US2020/012542**

(87) International publication number:
**WO 2020/146355 (16.07.2020 Gazette 2020/29)**

# (54) TREATMENT OF CANCER HAVING GNAQ OR GNA11 GENETIC MUTATIONS WITH PROTEIN KINASE C INHIBITORS

BEHANDLUNG VON KREBS MIT GENETISCHEN GNAQ- ODER GNA11-MUTATIONEN MIT PROTEINKINASE -C-INHIBITOREN

TRAITEMENT DU CANCER PRÉSENTANT DES MUTATIONS GÉNÉTIQUES GNAQ OU GNA11 AVEC DES INHIBITEURS DE PROTÉINE KINASE C

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2019 US 201962789177 P**

(43) Date of publication of application:
**17.11.2021 Bulletin 2021/46**

(73) Proprietor: **Ideaya Biosciences, Inc.**
**South San Francisco, CA 94080 (US)**

(72) Inventors:
- **KNOX, John**
**Emerald Hills, CA 94062 (US)**
- **LACKNER, Mark**
**San Mateo, CA 94403 (US)**
- **MOUNIR, Zineb**
**Pacifica, CA 94044 (US)**
- **O'BRIEN, Carol**
**Brisbane, CA 94005 (US)**

(74) Representative: **Harris, Jennifer Lucy et al**
**Kilburn & Strode LLP**
**Lacon London**
**84 Theobalds Road**
**London WC1X 8NL (GB)**

(56) References cited:
**WO-A1-2016/020864** **WO-A2-2011/130691**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 908 282 B1

## Description

## CROSS-REFERENCE TO RELATED APPLICATION

This application claims the benefit of and priority to U.S. Provisional Patent Application No. 62/789,177, filed January 07, 2019.

## BACKGROUND

Heterotrimeric guanine nucleotide-binding proteins (G proteins) couple to G Protein-couple Receptors (GPCRs), and serve as molecular switches that relay signals from activated GPCRs to a wide variety of intracellular effectors. G proteins represent a large family of heterotrimeric proteins found in mammals composed of alpha ($G\alpha$), beta ($G\beta$), and gamma ($G\gamma$) subunits. In their inactive state, G proteins are heterotrimeric, consisting of one $G\alpha$, one $G\beta$, and one $G\gamma$ subunit, and a bound deoxyguanosine diphosphate (GDP). Receptor-catalyzed guanine nucleotide exchange results in deoxyguanosine triphosphate (GTP) binding to the $G\alpha$ subunit and G protein activation. $G\alpha$-GTP dissociates from the $G\beta$ and $G\gamma$ subunits, allowing the $G\beta\gamma$ dimer and the $G\alpha$-GTP subunit each to activate downstream effectors. Hydrolysis of GTP to GDP deactivates the complex and turns off the cellular response.

Guanine Nucleotide-Binding Protein Alpha-Q (*GNAQ*) and Guanine Nucleotide-Binding Protein Alpha 11 (*GNA11*) are genes that encode $G\alpha_q$ and $G\alpha_{11}$ subunits respectively. The majority of somatic activating mutations found in *GNAQ* and *GNA11* result in constitutively active $G\alpha_q$ or $G\alpha_{11}$ proteins, respectively. Uveal melanoma, arising from melanocytes and biologically distinct from cutaneous melanomas, has been shown to have these activating mutations in *GNAQ* and/or *GNA11* genes. *GNAQ* and *GNA11* alterations at Q209 have been characterized as gain of function mutations that confer dependence on downstream Protein Kinase C (PKC) signaling. PKC is a family of multifunctional isoenzymes that plays a vital role in the regulation of signal transduction, cell proliferation and differentiation through positive and negative regulation of the cell cycle. PKC is one of the major downstream effectors in a $G\alpha_q$ ($G\alpha_{11}$ or $G\alpha_q/_{11}$) signaling pathway. The international patent applications WO 2011/130691 and WO 2016/020864 disclose the use of PKC inhibitors for the treatment of uveal melanoma carrying GNAQ or GNA11 mutations.

There is an unmet need for effective and safe therapeutic agents that can treat cancers other than uveal melanoma. The clinical significance and frequency of GNAQ and GNA11 mutations in non-uveal melanoma and other cancers is generally unknown and not understood.

## SUMMARY

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention. The present disclosure is based, in part, on the discovery that certain cancers are associated with a *GNAQ* and/or *GNA11* genetic mutation, and for example, once identified, such cancers may be treated by administering a pharmaceutically effective amount of a protein kinase C inhibitor. For example, provided herein is a PKC inhibitor for use in a method of treating a patient having a non-uveal melanoma tumor or cancer, or non-melanocytic tumor of the central nervous system, (e.g. pancreatic cancer, colorectal cancer, lung adenocarcinoma, cutaneous melanoma, stomach cancer, cervical cancer, uterine cancer, bladder cancer, hepatocellular carcinoma, prostate cancer, breast cancer, head and neck cancer, and glioblastoma), the method comprising: determining if the non-uveal melanoma or non-melanocytic tumor of the central nervous system has a *GNAQ* or *GNA11* genetic mutation that activates the PKC pathway; and orally administering to the patient suffering from the non-uveal melanoma cancer with *GNAQ or GNA11* genetic mutation a pharmaceutically effective amount of a protein kinase C inhibitor. Such determining may include determining if the non-uveal melanoma or non-melanocytic tumor of the central nervous system has a *GNAQ* Q209, *GNA11* Q209, *GNAQ* R183 or *GNA11* R183 genetic mutation, where for example, the non-uveal melanoma cancer or non-uveal melanoma tumor or non-melanocytic tumor of the central nervous system may selected from the group consisting of a pancreatic cancer, colorectal cancer, lung adenocarcinoma, and cutaneous melanoma. Alternatively, such determining may include determining if the non-uveal melanoma or non-melanocytic tumor of the central nervous system has a mutation other than any of *GNAQ* Q209, *GNA11* Q209, *GNAQ* R183 or *GNA11* R183 genetic mutation, and for example, the tumor or cancer may be selected from the group consisting of uterine, stomach, bladder, cervical, breast, head and neck, hepatocellular carcinoma, and glioblastoma.

For example, the present disclosure relates in a part to a PKC inhibitor for use in a method of treating a carcinoma, *e.g.,* a non-uveal melanoma solid tumor, having a *GNAQ* or *GNA11* genetic mutation (*e.g., a GNAQ* Q209, *GNAQ* R183, or a *GNA11* Q209 genetic mutation), or a non-Q209 or non-R183 mutation, in a patient in need thereof where the carcinoma for example, is selected from the group consisting of pancreatic adenocarcinoma, stomach adenocarcinoma, cervical

carcinoma, lung adenocarcinoma and cutaneous melanoma. In embodiments of the disclosure, such contemplated method may include determining if the patient's carcinoma has a *GNAQ* or *GNA11* genetic mutation; and orally administering to the patient having the carcinoma with the *GNAQ* or *GNA11* genetic mutation a therapeutically effective amount of a protein kinase C inhibitor, for example, a small molecule protein kinase C inhibitor.

Provided herein, for example, is a PKC inhibitor for use in a method of treating a solid tumor cancer in a patient in need thereof, the method comprising: orally administering an effective amount of a protein kinase C inhibitor to the patient, wherein the patient has been determined to have a *GNAQ* or *GNA11* genetic tumor mutation, wherein the solid tumor cancer is selected from pancreatic adenocarcinoma, stomach adenocarcinoma, cervical carcinoma, and lung adenocarcinoma.

For example, described herein is a PKC inhibitor for use in a method of treating a solid tumor cancer having a *GNAQ* or *GNA11* genetic mutation in a patient, for example, a patient in need thereof wherein the method includes determining if the solid tumor cancer has a *GNAQ* or *GNA11* genetic mutation; determining if the patient does not have activating *BRAF* or *NRAS* mutations (*e.g.,* the patient may not have a BRAF V600 mutation); and orally administering to the patient without activating *BRAF* or *NRAS* mutations a pharmaceutically effective amount of a protein kinase C inhibitor.

In some embodiments, contemplated methods include treating a solid tumor cancer in a patient in need thereof, where the patient is substantially non-responsive to treatment with an immune checkpoint inhibitor, *e.g.*, not responsive to a 3-month course of treatment with an immune checkpoint inhibitor such as one or more of pembrolizumab, ipilimumab, nivolumab, and atezolizumab, comprising orally administering to the patient a pharmaceutically acceptable amount of a protein kinase inhibitor, wherein the patient's solid tumor has been determined to have *e.g.,* a *GNAQ* or GNA11 mutation, *e.g.,* a Q209L or a *GNA11* Q209L genetic mutation.

Contemplated methods of treatment herein may be directed to patients who have a low tumor mutation burden. For example, in some embodiments the patient may have a low tumor load of mutations in one or more of *BAP1, SF3B1, EIF1AX, TERT, BRAF, CDKN2A, NRAS,* or *KRAS.*

## BRIEF DESCRIPTION OF FIGURES

FIGURE 1 depicts Galpha-q with mutations mapped in CPK and GTP binding site in background surface.

FIGURE 2 depicts GNA11 Homology model with mutations mapped in CPK and putative GTP binding site in background surface.

FIGURE 3 is a schematic diagram of the study described in Example 3. Abbreviations used in the figure: BID: bis in diem/twice a day; MUM: metastatic uveal melanoma; CRC: colorectal cancer; RP2D: recommended phase 2 dose.

## DETAILED DESCRIPTION

The present disclosure is directed, in part, to PKC inhibitors for use in methods for treating cancer in patients that have mutations, for example, activating mutations that for example, activate the PKC pathway, in *GNAQ* or *GNA11,* wherein the method includes orally administering a PKC inhibitor such as disclosed herein. The disclosure described herein is useful for the treatment of non-uveal melanoma cancers as defined in the claims that have either an activating mutation of *GNAQ* or *GNA11 e.g.,* heterozygous somatic substitutions of Q209 or R183 of *GNAQ* or *GNA11,* or have an activating mutation other than each of a Q209 or R183 mutation, by orally administering to a patient in need thereof a protein kinase C inhibitor.

### Definitions

By "treating" is meant reducing at least one symptom associated with the disease or condition being treated.

A "subject" or "patient" as described herein, refers to any animal at risk for, suffering from or diagnosed for cancer (for example, a carcinoma, a solid tumor cancer, or a non-uveal melanoma tumor or a non-melanocytic tumor of the central nervous system), including, but not limited to, mammals, primates, and humans. In certain embodiments, the subject may be a non-human mammal such as, for example, a cat, a dog, or a horse. In another embodiment, the subject is a human subject. A subject may be an individual diagnosed with a high risk of developing cancer, someone who has been diagnosed with cancer, someone who previously suffered from cancer, and/or an individual evaluated for symptoms or indications of cancer.

The terms "polypeptide," "peptide" and "protein" are used interchangeably herein to refer to a polymer of amino acid residues. The terms apply to amino acid polymers in which one or more amino acid residue is a non-natural chemical

mimetic of a corresponding naturally occurring amino acid, as well as to naturally occurring amino acid polymers, those containing modified amino acid residues, and non-naturally occurring amino acid polymer.

As used herein, the term "pharmaceutically acceptable salts" refers to the nontoxic acid or alkaline earth metal salts of compounds described herein, for example, compounds of Formula (II). These salts can be prepared *in situ* during the final isolation and purification of the compounds, for example, the compounds of Formula (II), or by separately reacting a base or acid functional group of compounds described herein, for example, compounds of Formula (II), with a suitable organic or inorganic acid or base, respectively. Representative salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, pamoate, pectinate, persulfate, 3-phenylproionate, picrate, pivalate, propionate, succinate, sulfate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

As used herein, the term *"GNAQ"* refers to Guanine Nucleotide-Binding Protein Alpha-Q gene that encodes the Gq alpha subunit (G$\alpha$q) and the term "*GNA11*" refers to Guanine Nucleotide-Binding Protein Alpha 11 genes that encodes the G11alpha subunit (G$\alpha$11) subunit. The term encompasses nucleic acid and polymorphic variants, alleles, mutants, and fragments of GNAQ and GNA11. *GNAQ* and *GNA11* sequences are well known in the art. Examples of human GNAQ sequences are available under the reference sequences NM_002072 in the NCBI nucleotide database (nucleotide sequence) and accession number NP_002063.2 (polypeptide sequence). Human GNAQ has been localized to chromosome 9q21. Examples of human GNA11 sequences are available under the reference sequences NM_002067 in the NCBI nucleotide database (nucleotide sequence) and accession number NP_002058.2 (polypeptide sequence). Human GNA11 is localized to chromosome region 19p13.3.

As used herein, "mutations" can refer to changes in a polynucleotide sequence that result in changes to protein activity. Mutations can be nucleotide substitutions, such as single nucleotide substitutions, insertions, or deletions. *GNAQ* and *GNA11* mutations are typically activating mutations that lead to constitutive activation of the $\alpha$ subunit. Without being bound to a theory, it is believed that the constitutive activity results from a lack of the GTP-hydrolase activity in the mutant *GNAQ* or *GNA11* protein. Activating mutations can also refer to mutations that result in a loss or decrease of GTP hydrolyzing activity of a G$\alpha$ subunit. Mutations in *GNAQ* and *GNA11* include a substitution of arginine in codon R183 or substitution of glutamine in codon Q209, or may be other mutations. In an embodiments, mutations in *GNAQ* and/or *GNA11* can be selected from group comprising of: Q209P, Q209L, Q209H, Q209K, Q209Y, Q209R, Q209H, R183Q, R183, for example, GNAQ Q209 may be mutated to either P or L as well as to R or H; GNAQ R183 may be mutated to Q; GNA11 Q209 may be mutated to L as well as to P or K; GNAQ R183 may mutate to C or H.

GNA11 Q209 can be mutated to L as well as rarely to P or K; also GNAQ R183 is most often mutate to C and more rarely to H.

As used herein, the term "PKC" refers to protein kinase C. The PKC family of serine/threonine kinases is composed of at least ten isoforms, pivotal in various cellular differentiation processes with distinctive means of regulation and tissue distribution Five isozymes are known to be present in human neutrophils (*see* Karlsson A. et al (2002) antioxid. Redox Signal. 4:49-60).

As used herein, the term "PKC inhibitor" refers to a protein kinase C inhibitor that may be pan (multi-subtype) or selective to one or more PKC isozymes. The term PKC generally refers to the entire family of isoforms: conventional isoforms; alpha, beta, and gamma, novel isoforms; delta, epsilon, eta, and theta, and atypical isoforms; zeta, and iota. The term "inhibitor" refers to modulatory molecules or compounds that, *e.g.,* bind to, partially or totally block activity, decrease, prevent, delay activation, inactivate, desensitize, or down regulate the activity or expression of a *e.g.,* a protein, *e.g.,* PKC.

The phrase "alkyl" refers to alkyl groups that do not contain heteroatoms. Thus, the phrase includes straight chain alkyl groups, such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example: $-CH(CH_3)_2$, $-CH(CH_3)(CH_2CH_3)$, $-CH(CH_2CH_3)_2$, $-C(CH_3)_3$, $-C(CH_2CH_3)_3$, $-CH_2CH(CH_3)_2$, $-CH_2CH(CH_3)(CH_2CH_3)$, $-CH_2CH(CH_2CH_3)_2$, $-CH_2C(CH_3)_3$,$-CH_2C(CH_2CH_3)_3$, $-CH(CH_3)-CH(CH_3)(CH_2CH_3)$, $-CH_2CH_2-CH(CH_3)_2$, $-CH_2CH_2CH(CH_3)(CH_2CH_3)$, $-CH_2CH_2CH(CH_2CH_3)_2$, $-CH_2CH_2C(CH_3)_3$, $-CH_2CH_2C(CH_2CH_3)_3$, $-CH(CH_3)CH_2-CH(CH_3)_2$, $-CH(CH_3)CH(CH_3)CH(CH_3)_2$, $-CH(CH_2CH_3)CH(CH_3)CH(CH_3)(CH_2CH_3)$, and others. The phrase also includes cyclic alkyl groups such as cyclopropyl, cyclobutyl, cyclopentyl, cyclohexyl, cycloheptyl, and cyclooctyl, and such rings substituted with straight and branched chain alkyl groups as defined above. Thus, the term "$C_{1-12}$ alkyl group" includes primary alkyl groups, secondary alkyl groups, and tertiary alkyl groups. Alkyl groups include straight and branched chain alkyl groups and cyclic alkyl groups having 1 to 12 carbon atoms.

As used herein, "$C_{1-6}$ alkyl" includes both substituted or unsubstituted straight or branched chain alkyl groups

having from 1 to 6 carbon atoms. Representative $C_{1-6}$ alkyl groups include, for example, methyl, ethyl, propyl, isopropyl, n-butyl, tert-butyl, neopentyl, trifluoromethyl, pentafluoroethyl and the like. Unless stated otherwise, $C_{1-6}$ alkyl groups may be substituted, such as with halo, hydroxy, amino, nitro and/or cyano groups, and the like. Representative $C_{1-3}$ haloalkyl and $C_{1-3}$ hydroxyalkyl include chloromethyl, trichloromethyl, trifluoromethyl, fluoromethyl, fluoroethyl, chloroethyl, hydroxymethyl, hydroxyethyl, and the like. Other suitable substituted $C_{1-3}$ alkyl moieties include, for example, aralkyl, aminoalkyl, aminoaralkyl, carbonylaminoalkyl, alkylcarbonylaminoalkyl, arylcarbonylaminoalkyl, aralkylcarbonylaminoalkyl, aminoalkoxyalkyl and arylaminoalkyl, unless stated otherwise.

As used herein, "$C_{1-6}$ alkoxy" as used herein refers to the radical RO-, wherein R is $C_{1-6}$ alkyl. Representative examples of $C_{1-6}$ alkoxy groups include methoxy, ethoxy, t-butoxy, trifluoromethoxy and the like.

As used herein, the term "halogen" or "halo" refers to chloro, bromo, fluoro and iodo groups.

"Haloalkyl" refers to a $C_{1-3}$ alkyl radical substituted with one or more halogen atoms.

The term "haloalkoxy" refers to a $C_{1-3}$ alkoxy radical substituted with one or more halogen atoms.

Hydroxy refers to the group -OH.

"Amino" refers herein to the group $-NH_2$.

The term "$C_{1-3}$ alkylamino" refers herein to the group -NRR' where R and R' are each independently selected from hydrogen or a $C_{1-3}$ alkyl provided at least one of R and R' is $C_{1-3}$ alkyl.

The term "arylamino" refers herein to the group -NRR' where R is $C_{6-10}$ aryl, including phenyl, and R' is hydrogen, a $C_{1-3}$ alkyl, or $C_{6-10}$ aryl, including phenyl.

The term "$C_{3-8}$ cycloalkyl" refers to a mono- or polycyclic, heterocyclic or carbocyclic $C_{3-8}$ alkyl substituent. Typical cycloalkyl substituents have from 3 to 8 backbone (*i.e.,* ring) atoms in which each backbone atom is either carbon or a heteroatom. The term "heterocycloalkyl" refers herein to cycloalkyl substituents that have from 1 to 5, and more typically from 1 to 4 heteroatoms in the ring structure. Suitable heteroatoms employed in compounds of the present disclosure are nitrogen, oxygen, and sulfur. Representative heterocycloalkyl moieties include, for example, morpholino, piperazinyl, piperidinyl and the like. Carbocycloalkyl groups are cycloalkyl groups in which all ring atoms are carbon. When used in connection with cycloalkyl substituents, the term "polycyclic" refers herein to fused and non-fused cyclic alkyl structures. The term "carbobicyclic or carbobicyclyl" refers to a saturated, or partially unsaturated carbocyclic ring fused to another carbocyclic ring, aryl ring, heterocyclic ring or heteroaryl ring. The cycloalkyl group is unsubstituted or substituted.

The term "heterocycle" or "heterocyclyl" includes rings in which nitrogen is the heteroatom as well as partially and fully-saturated rings. Exemplary heterocycles include but are not limited to, for example: piperidinyl, piperazinyl, 1,2-oxazinane, 2-oxopiperazinyl, 2-oxopiperidinyl, N-methyl piperazinyl, and morpholinyl, each optionally substituted.

Heterocyclic moieties can be unsubstituted or monosubstituted or disubstituted with various substituents independently selected from hydroxy, halo, oxo (C=O), alkylimino (RN=, wherein R is a $C_{1-3}$ alkyl or $C_{1-3}$ alkoxy group), amino, $C_{1-3}$ alkylamino, $C_{1-3}$ dialkylamino, acylaminoalkyl, $C_{1-3}$ alkoxy, $C_{1-3}$ alkyl, cycloalkyl or $C_{1-3}$ haloalkyl. Heterocyclic groups (heterocyclyl) may be attached at various positions as will be apparent to those having skill in the organic and medicinal chemistry arts in conjunction with the herein.

The term "heteroaryl" refers to 5-10 membered carbocyclic ring system, including fused ring systems, having 1 to 4 heteroatoms each independently selected from the group consisting of: O, N and S. Said heteroaryl may be optionally substituted with one or two substituents. The term "heteroaryl" also refers herein to $C_{6-10}$ aryl groups having from 1 to 4 heteroatoms as ring atoms in an aromatic ring with the remainder of the ring atoms being carbon atoms. Exemplary substituents include, but are not limited to: halo, CN, $C_{1-3}$ alkyl, $C_{1-3}$ alkoxy, $C_{1-3}$ haloalkyl, $C_{1-3}$ haloalkoxy, $C_{3-7}$ cycloalkyl, and 4-7 membered heterocyclyl having 1 or 2 heteroatoms selected from N, O and S, said heterocyclyl optionally substituted with 1 to 3 substituents each independently selected from the group consisting of: halo, CN, $C_{1-3}$ alkyl, $C_{1-3}$ alkoxy, $C_{1-3}$ haloalkyl, and $C_{1-3}$ haloalkoxy. Representative heteroaryl groups include, for example, those shown below. Representative heteroaryls include, for example, imidazolyl, pyridinyl (also referred to aspyridyl), pyrazinyl, azetidinyl, thiazolyl, triazolyl, benzimidazolyl, benzothiazolyl, thiazolyl, thiazolidinyl, isothiazolyl, isothiazolidinyl, indolyl, quinolinyl, isoquinolinyl, azetidinyl, N-methylazetidinyl, pyrimidinyl, pyridazinyl, oxazolyl, oxazolidinyl, isoxazolyl, isoazolidinyl, benzimidazolyl, benzothiazolyl, benzoxazolyl, furyl, thienyl, triazolyl, benzothienyl diazapinyl, pyrryl, pyrrolinyl, pyrrolidinyl, pyrazolyl, pyrazolinyl, pyrazolidinyl, imidazoyl, imidazolinyl, imidazolidinyl and benzoxazolyl. The heteroaryl is unsubstituted or substituted with 1 to 3 substituents each independently selected from the group consisting of: H, $^2$H, halo, $C_{2-3}$ alkynyl, $C_{2-3}$ alkenyl, CN, $C_{1-3}$ alkyl, $C_{1-3}$ alkoxy, $C_{1-3}$ haloalkyl, $C_{1-3}$ haloalkoxy, $C_{3-7}$ cycloalkyl, $CONH_2$, $CONHC_{1-3}$ alkyl, $CONHC_{6-10}$ aryl, $SO_2NH_2$, $SO_2NHC_{1-3}$ alkyl, $SO_2NHC_{6-10}$ aryl and 4-7 membered heterocyclyl having 1 to 3 heteroatoms selected from N, O and S, said heterocyclyl optionally substituted one or two substituents each independently selected from the group consisting of: H, $^2$H, halo, CN, $C_{1-3}$ alkyl, $C_{1-3}$ alkoxy, $C_{1-3}$ haloalkyl, and $C_{1-3}$ haloalkoxy.

The term "$^2$H" refers to a heavy isotope of hydrogen that is also referred to as deuterium (D). It is understood that the above definitions are not intended to include impermissible substitution patterns (*e.g.*, methyl substituted with five fluoro groups or a halogen atom substituted with another halogen atom).

As used herein, the term "MEK inhibitor" refers to a mitogen-activated protein kinase (MEK) inhibitor. The term "inhibitor" refers to modulatory molecules or compounds that, *e.g.,* bind to, partially or totally block activity, decrease,

prevent, delay activation, inactivate, desensitize, or down regulate the activity or expression of MEK, mTOR, or $CDK_i$. Representative MEK inhibitors include trametinib, cobimetinib, binimetinib, and selumetinib as well as TAK733, PD-325901, CI-1040, PD 184352, and PD035901. A representative mTor inhibitor includes rapamycin. $CDK_i$ (cyclin dependent kinase inhibitors) include ribociclib, palbociclib, abemaciclib, abernaciclib, ribociclib, and trilaciclib.

As used herein, the term "HDM2-p53 inhibitor" refers to human double minute 2 protein inhibitors, regulators or modulators, pharmaceutical compositions containing the compounds and potential methods of treatment using the compounds and compositions to potentially treat diseases such as, for example, cancer, diseases involving abnormal cell proliferation, and include small molecule inhibitors HDM201 (siremaldin), and JNJ-26854165.

As used herein, the term "mutational load" refers to the level, *e.g.*, number, of an alteration (*e.g.,* one or more alterations, *e.g.,* one or more somatic alterations) per a preselected unit (*e.g.,* per megabase) in a predetermined set of genes or all analyzed genes (*e.g.,* in the coding regions of the predetermined set of genes). Mutation load can be measured, *e.g.*, on a whole genome or exome basis, or on the basis of a subset of genome or exome. In certain embodiments, the mutation load measured on the basis of a subset of genome or exome can be extrapolated to determine a whole genome or exome mutation load. The terms "mutation load," "mutational load," "mutation burden," and "mutational burden" are used interchangeably herein. In the context of a tumor, a mutational load is also referred to herein as "tumor mutational burden," "tumor mutation burden," or "TMB."

As used herein, the term "*BAP1*" refers to BRCA1-associated protein-1 gene (ubiquitin carboxy-terminal hydrolase; BAP1). The nucleic acid and amino acid sequences of BAP1 are known and publicly available (Genbank NM_004656.2, Genbank NP_004647.1). BAP1 has been functionally implicated in the DNA damage response as well as in the regulation of apoptosis, senescence and the cell cycle. Deletions and inactivating mutations in BAP1 have been previously associated with tumors of the breast and lung and, consistent with BAP1's role as tumor suppressor, restoration of BAP1 function has been shown to suppress cell growth and tumorigenicity in a BAP1-mutant lung cancer cell line.

As used herein, "SF3B1" refers to a gene that encodes Splicing Factor 3b Subunit 1. The nucleic acid and amino acid sequences of SF3B1 are known and publicly available (NM_012433.3, Genbank NP_036565.2). Subunit 1 of the splicing factor 3b protein complex plays a number of critical roles in the splicing mechanism of the cell. Mutations in SF3B1 affect the ability of a cell to convert pre-mRNA, which contains intronic sequence, into mature mRNA.

As used herein, *"E1F1AX*" refers to a gene that encodes for the protein X-linked eukaryotic translation initiation factor 1A, which plays a role in protein synthesis. The nucleic acid and amino acid sequences of E1F1AX are known and publicly available (NM_001412.4, Genbank NP_001403.1). *E1F1AX* is commonly mutated in uveal melanoma.

As used herein, "TERT" can refer to either the gene encoding the enzyme Telomerase Reverse Transcriptase (TERT) or to the enzyme (i.e., protein) itself. TERT refers to the nucleoprotein, or enzyme, portion of telomerase. TERT genes have also been called "Ever Shorter Telomeres" or "EST" genes. Mutations in the promoter region of TERT have been associated with cancers including, but not limited to, thyroid cancer, bladder cancer and glioblastoma. The nucleic acid and amino acid sequences of TERT are known and publicly available (NM_198253.2, Genbank NP_937983.2).

As used herein, "NRAS" or "neuroblastoma RAS viral oncogene homolog" refer to a small GTPase Ras family protein encoded on chromosome 1. The nucleic acid and amino acid sequences of NRAS are known and publicly available (NM_002524, Genbank NP_002515).

As used herein, "BRAF" or "v-Raf murine sarcoma viral oncogene homolog B" refer to a Raf kinase family serine/threonine-specific protein kinase that interacts with AKT1; CRaf, HRAS, and YWHAB. The sequences of BRAF are well known in the art for a number of species, *e.g.,* human BRAF (NM_004333, Genbank NP_004324). the NRAS and/or BRAF sequence variation(s) can be point mutations. In some embodiments, a NRAS point mutation can be a point mutation resulting in one of the following amino acid residue changes: G12D; G12S; G13A; G13C; G13D; G12R; G13V; Q61H1; Q61K; Q61L; Q61R1; and Q61R2. In some embodiments, a BRAF point mutation can be a point mutation resulting in one of the following amino acid residue changes: V600D TG/AT; V600E T/A; V600E TG/AA; and V600K GT/AA.

As used herein, the terms 'solid tumors' and 'solid tumor cancers' may be used interchangeably. Provided herein, for example, is a method of treating a patient having a non-uveal melanoma tumor or non-melanocytic tumor of the central nervous system, comprising: determining if the non-uveal melanoma or non-melanocytic tumor of the central nervous system has a *GNAQ* or *GNA11* genetic mutation that activates the PKC pathway; and orally administering to the patient suffering from the non-uveal melanoma cancer with *GNAQ* or *GNA11* genetic mutation a pharmaceutically effective amount of a protein kinase C inhibitor, such as one described herein. Determining may include determining if the non-uveal melanoma or non-melanocytic tumor of the central nervous system has a *GNAQ* Q209, GNA11 Q209, *GNAQ* R183 or *GNA11* R183 genetic mutation Such mutations may be present for example, in a contemplated method of treating non-uveal melanoma cancer or non-uveal melanoma tumor or non-melanocytic tumor of the central nervous system selected from the group consisting of a pancreatic cancer tumor, colorectal cancer tumor, lung adenocarcinoma, and cutaneous melanoma, in a patient in need thereof.

Alternatively, determining may include determining if the non-uveal melanoma or non-melanocytic tumor of the central nervous system has a mutation other than any of *GNAQ* Q209, *GNA11* Q209, *GNAQ* R183 or *GNA11* R183 genetic mutation, such as another mutation provided herein. Such mutations may be present in a non-uveal melanoma cancer or

non-uveal melanoma tumor or non-melanocytic tumor of the central nervous system selected from the group consisting of uterine, stomach, cervical, bladder, hepatocellular carcinoma, prostate, breast, head and neck, or glioblastoma cancers/tumors. For example, contemplated methods may be used to treat patients having a non-uveal tumor that may have a different mutation in GNAQ or GNA11 other a Q209 or R183 mutation that e.g., activates the PKC pathway in a specific cancer such as *e.g.,* uterine, stomach, cervical, bladder, hepatocellular carcinoma, prostate, breast, head and neck, or glioblastoma cancer.

Provided herein, in one embodiment, is a PKC inhibitor for use in a method of treating a patient having a non-uveal melanoma tumor or non-melanocytic tumor of the central nervous system, the method comprising determining if the non-uveal melanoma or non-melanocytic tumor of the central nervous system has a *GNAQ* or GNA11 mutation, *e.g., a GNAQ* Q209L or GNA11 Q209L genetic mutation; and orally administering to the patient suffering from the non-uveal melanoma cancer with the *GNAQ* or *GNA11* genetic mutation a pharmaceutically effective amount of a protein kinase C inhibitor. Such non-uveal melanoma cancer or non-uveal melanoma tumor or non-melanocytic tumor of the central nervous system may include pancreatic cancer tumor, stomach cancer tumor, colorectal cancer tumor, cervical cancer tumor, lung adenocarcinoma, and cutaneous melanoma, uterine, stomach, cervical, bladder, hepatocellular carcinoma, prostate, breast, head and neck, or glioblastoma cancer/tumors.

For example, a PKC inhibitor for use in a method of treating a carcinoma having a *GNAQ* or *GNA11* genetic mutation in a patient in need thereof is provided, wherein the carcinoma is selected from the group consisting of pancreatic adenocarcinoma, stomach adenocarcinoma, cervical carcinoma, and lung adenocarcinoma, the method comprising determining if the carcinoma has a *GNAQ* or *GNA11* genetic mutation; and orally administering to the patient having the carcinoma with the *GNAQ* or *GNA11* genetic mutation a therapeutically effective amount of a protein kinase C inhibitor.

In another embodiment, a PKC inhibitor for use in a method of treating a solid tumor cancer in a patient in need thereof is provided the method, comprising orally administering an effective amount of a protein kinase C inhibitor to the patient, wherein the patient has been determined to have a *GNAQ* or *GNA11* genetic tumor mutation, wherein the solid tumor cancer is selected from pancreatic adenocarcinoma, stomach adenocarcinoma, cervical carcinoma, and lung adenocarcinoma.

In some embodiments of the methods disclosed herein, the *GNAQ* or *GNA11* mutation can be any one of a number of mutations, including a substitution mutation, an insertion mutation, and/or a deletion mutation. In some embodiments, the *GNAQ* or *GNA11* mutation is a gain of function mutation. In some embodiments, the *GNAQ* or *GNA11* mutation is the substitution of glutamine in codon 209 (Q209) and/or a substitution of arginine in codon R183. In some embodiments, the *GNAQ* mutation is one of Q209P, Q209L, Q209H, Q209K, or Q209Y. Also as noted above, in some embodiments the carcinoma, the cancer, the solid tumor cancer, or the non-uveal melanoma tumor or non-melanocyctic tumor of the central nervous system has a *GNA11* mutation. For example, in embodiments described herein, the *GNA11* mutation is one of Q209P, Q209L, Q209K or Q209H. For example, in particular embodiments, the *GNAQ* or *GNA11* mutation is Q209L. In some embodiments, the *GNAQ* or *GNA11* mutation is the substitution of arginine in codon R183. For example, in particular embodiments, the *GNAQ* mutation is R183Q, or the *GNA11* mutation is R183C or R183H. Alternatively, the *GNAQ* or GNA11 mutation is at R256, L279, R166, A168, R210, R213, R166, A231, A342, D333, G171, R147, R73, T47, E191, E221, R149, T175, T379, T85, A86, E163, D195, E319, E191, E280, E49, P293, R300, R338, R60, D155, D205, D321, I226, R37, or V240.

Determining if a patient (*e.g.,* the carcinoma or tumor) has a *GNAQ* or *GNA11* genetic mutation may include identifying *GNAQ* or *GNA11* mutations in DNA extracted from a tumor sample and/or in circulating tumor or tumor cell DNA.

For example, provided herein is a PKC inhibitor for use in a method of treating a non-uveal solid tumor cancer having a GNAQ or GNA11 genetic mutation in a patient in need thereof, the method comprising: determining if the solid tumor cancer has a *GNAQ* or *GNA11* genetic mutation; determining if the patient does not have activating BRAF or NRAS mutations (e.g. does not have a BRAF V600 mutation); and orally administering to the patient without activating BRAF or NRAS mutations a pharmaceutically effective amount of a protein kinase C inhibitor. Such solid tumor cancers may be selected from the group consisting of pancreatic cancer, stomach cancer, colorectal cancer, cervical cancer, lung adenocarcinoma, cutaneous melanoma, uterine, bladder, hepatocellular carcinoma, prostate, breast, head and neck, and glioblastoma.

In another embodiment, a PKC inhibitor for use in a method of treating a non-uveal solid tumor cancer in a patient in need thereof is provided, wherein the patient is substantially non-responsive to treatment with an immune checkpoint inhibitor, comprising orally administering to the patient a pharmaceutically effective amount of a protein kinase C inhibitor, wherein the patient's solid tumor has been determined to have a *GNAQ* or *GNA11* genetic mutation.

For example, the *GNAQ* or *GNA11* mutation may be the substitution of glutamine in codon 209 (Q209) or wherein the *GNAQ* or *GNA11* mutation is the substitution of arginine in codon R183, and for example, the solid tumor is one of cutaneous melanoma, colorectal, lung adenocarcinoma, or pancreatic. Alternatively, the *GNAQ* or GNA11 mutation is other than a substitution of glutamine in codon 209 (Q209) and other than a substitution of arginine in codon R183, and the solid tumor may be, *e.g.,* one of uterine, stomach, cervical, hepatocellular carcinoma, prostate, breast, head and neck or glioblastoma.

The disclosed methods may include treating patients with low tumor mutational burden of less than 10 mut/Mb, 11 mut/Mb, 12 mut/Mb, 13 mut/Mb, 14 mut/Mb, 15 mut/Mb, 16 mut/Mb, 17 mut/Mb, 18 mut/Mb, 19 mut/Mb or 20 mut/Mb, for example, a low tumor mutational load of mutations in one or more of *BAP1, SF3B1, EIF1AX, TERT, BRAF, KRAS,* and/or *NRAS.* For example, the patient may have a low tumor mutational burden of less than 17 mut/Mb or of less than 10 mut/Mb, for example, the patient may have a low tumor mutational load of mutations across the genome, *e.g.,* one or more of BAP1, *SF3B1,* EIF1AX and TERT. In certain embodiments, contemplated patients may not have activating BRAF or NRAS mutations. In certain embodiments, disclosed methods are directed to patients that do not have a BRAF V600 mutation.

In other aspects, the present disclosure provides a PKC inhibitor for use in a method for treating cancers with mutations in *GNAQ* and/or *GNA11,* for example, activating *GNAQ* or *GNA11* mutations, in a human or animal subject in recognized need of such treatment comprising administering to said subject an amount of a compound, for example, a compound of formula (II), or *e.g.,* a compound represented by Formula III effective to inhibit PKC activity in the subject.

Provided herein, in an embodiment, is a PKC inhibitor for use in a method of treating a patient having a non-uveal melanoma tumor or non-melanocytic tumor of the central nervous system, the method comprising: determining if the non-uveal melanoma or non-melanocytic tumor of the central nervous system has Q209L genetic mutation or a non Q209 mutation, and orally administering to the patient suffering from the non-uveal melanoma cancer with *GNAQ* Q209L or *GNA11* Q209L genetic mutation, or non Q209 mutation, a pharmaceutically effective amount of a protein kinase C inhibitor.

In other aspects, the present disclosure provides a PKC inhibitor for use in a method for treating non-uveal melanoma tumor or non-melanocytic tumor of the central nervous system harboring *GNAQ* or *GNA11* mutations in a human or animal subject in recognized need of such treatment. The method can include orally administering to the subject a therapeutically effective amount of a PKC inhibitor compound, for example, a compound of formula (II) or *e.g.,* a compound represented by Formula III.

In another aspect, the present disclosure provides a PKC inhibitor for use in a method for treating a patient that is or has been substantially non-responsive to treatment with an immune checkpoint inhibitor. The method can include orally administering to such a patient a therapeutically effective amount of a PKC inhibitor compound, for example, a compound of Formula (II), Immune checkpoint inhibitors to which a patient is or has been nonresponsive can include pembrolizumab, ipilimumab, nivolumab, cemiplimab, avelumab, durvalumab, and atezolizumab. The aforementioned patient can be a patient that is or has been non-responsive to a 1 month, 2 month, 3 month, 4 month, 5 month, 6 month, 1 year, or longer than 1 year course of treatment with an immune checkpoint inhibitor. For example, such a patient is not and/or has not been responsive to a 3-month course of treatment with an immune checkpoint inhibitor such as pembrolizumab, ipilimumab, nivolumab, and/or atezolizumab.

For example, described herein is a PKC inhibitor for use in a method of treating a cancer in patient in need thereof wherein the method includes: identifying the patient as having a tumor with a mutation that includes a substitution of arginine in codon R183 in GNAQ or GNA11; and orally administering a composition comprising a compound represented by Formula III, or a pharmaceutically acceptable salt thereof, to the patient; where the cancer is selected from the group consisting of pancreatic cancer tumor, stomach cancer tumor, colorectal cancer tumor, cervical cancer tumor, lung adenocarcinoma, and cutaneous melanoma.

Disclosed methods may include administering the protein kinase C inhibitor as a monotherapy or may further include administering a therapeutically effective amount of one or more additional therapeutic agents such as a mitogen-activated protein kinase (MEK) inhibitor, a mTOR inhibitor, a $CDK_i$ inhibitor, an immune checkpoint inhibitor, or a HDM2-p53 inhibitor. MEK inhibitors include trametinib, cobimetinib, binimetinib, and selumetinib. In some embodiments, methods described herein further include administering a therapeutically effective amount of a HDM2-p53 inhibitor.

Also provided herein is a PKC inhibitor for use in a method of treating a cancer in patient in need thereof, said method comprising: identifying the patient as having a tumor with a mutation of *GNAQ* or *GNA11*; orally administering a composition comprising a small molecule PKC inhibitor such as represented in Formula III to the patient; wherein the cancer is selected from the group consisting of pancreatic cancer tumor, stomach cancer tumor, colorectal cancer tumor, cervical cancer tumor, lung adenocarcinoma, cutaneous melanoma, colorectal cancer, stomach cancer, bladder cancer, hepatocellular carcinoma, prostate cancer, breast cancer, head and neck cancer, and glioblastoma.

## PKC Inhibitors

PKC inhibitors useful in the practice of the present disclosure may inhibit several isoforms of PKC, *e.g.,* may inhibit one or more of the isoforms α, β-1, β2, γ,δ, ε, η, θ, ζ, ι. In particular embodiments described herein, contemplated PKC inhibitors may inhibit a inhibit PKC α and θ isoforms, and/or the δ and/or ε isoforms. Suitable PKC inhibitors include those disclosed herein, or may include maleimide derivatives such as bisindoylmaleimide, enzastaurin, staurospoine, , roboxistaurin, sotrastaurin, rottlerin, and/midostaurin. In some embodiments, a contemplated PKC inhibitor is a small molecular protein kinase C inhibitor, which may for example, be orally administrable.

For example, protein kinase C (PKC) inhibitors contemplated herein can have potency against one or multiple PKC isoforms including, but not limited to, one or more of δ, ε, η, θ, and/or α PKC isoforms. For example, a contemplated

protein kinase C inhibitor for use in the disclosed methods may be a small molecule protein kinase C inhibitor that has potency against multiple protein kinase C informs. For example, a contemplated protein kinase C inhibitor of the present disclosure has potency against one or more (*e.g.*; one, two, three, four, five, six, seven or more) of δ, ε, η, θ, β, γ, α protein kinase C isoforms. For example, the protein kinase C inhibitor of the present disclosure may have a potency against two or more of δ, ε, η, θ, β, γ, or α protein kinase C isoforms. The protein kinase C inhibitor, of the present disclosure may, in an embodiment, may have a potency against each of δ, ε, η, θ, β, γ, or α protein kinase C isoforms. In yet another embodiment, the protein kinase C inhibitor has an $IC_{50}$ with respect to PKC θ/α and/or PKC δ and/or PKC ε isoforms of less than 50 nM For example, in some embodiments the PKC inhibitor has an $IC_{50}$ with respect to each of PKC θ/α, PKC δ, and/or PKC ε isoforms of independently selected, less than 100 nM, less than 90 nM, less than 80 nM, less than 70 nM, less than 60 nM, less than 50 nM, less than 40 nM, less than 30 nM, less than 20 nM, less than 10 nM, less than 5 nM, or in some embodiments, less than 1 nM.

Examples of PKC inhibitors suitable for use with the present disclosure include those contemplated herein, and may also include include staurosporine, the staurosporine analogue CPG41251, bryostatin-1, KAI-9803, 7-hydroxystaurosporine, L-threo-dihydrosphingosine (safingol), AHT956 and AEB071, the non-selective PKC inhibitor (PKC412), ilmofosine (BM 41 440), indolcarbazole Gö6796 which is a more specific inhibitor of the classical PKC isoforms including PKCμ, the PKC-alpha antisense inhibitor LY900003, and the PKC-beta inhibitors LY333531, LY317615 (Enzastaurin). An example of an antisense molecule suitable for use in depleting PKC-alpha mRNA is 5'-GTTCTCGCTGGTGAGTTTCA-3'.

In accordance with some aspects of the present disclosure, a contemplated PKC inhibitor may be represented by formula (II):

(II)

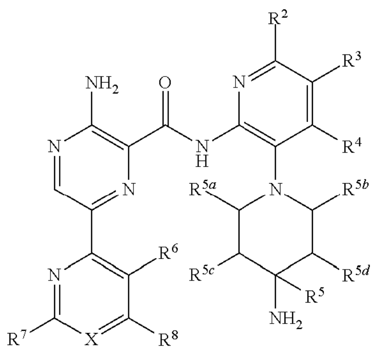

wherein:

X is N or CR;

R, $R^2$, $R^3$ and $R^4$ are each independently selected from the group consisting of H, $^2H$, halogen, hydroxyl (-OH), $C_{1-3}$ alkoxy, and $C_{1-3}$ alkyl; wherein $C_{1-3}$alkoxy may optionally be substituted by one, two, three or more halogens; and wherein $C_{1-3}$alkyl may optionally be substituted by one, two, three or more substituents, each independently selected from the group consisting of hydroxyl, halogen and $C_{1-3}$alkoxy (optionally substituted by one or more halogens);

$R^5$ is selected from the group consisting of -H, $^2H$, $CH_3$, $CH_2F$, $CHF_2$, $CF_3$, $CH_2OH$, and $C_{2-3}$ alkyl; wherein $C_{2-3}$alkyl may optionally be substituted by one, two, three or more substituents, each independently selected from the group consisting of fluorine, hydroxyl and $C_{1-3}$alkoxy (optionally substituted by one or more halogens);

$R^{5a}$ and $R^{5b}$ are each independently selected from the group consisting of H, $^2H$, and $C_{1-3}$ alkyl; wherein $C_{1-3}$alkyl may optionally be substituted by one, two, three or more substituents, each independently selected from the group consisting of fluorine, hydroxyl and $C_{1-3}$alkoxy; or $R^{5a}$ and $R^{5b}$ are taken together to form a methylene or ethylene bridging group;

$R^{5c}$ and $R^{5d}$ are each independently selected from the group consisting of H, $^2H$, F, - OH, $C_{1-3}$alkoxy, and $C_{1-3}$ alkyl; wherein $C_{1-3}$alkyl may optionally be substituted by one, two, three or more substituents, each independently selected from the group consisting of fluorine, hydroxyl and $C_{1-3}$alkoxy; or $R^{5c}$ and $R^{5d}$ taken together form a methylene, ethylene or $-CH_2-O-$ bridging group;

$R^6$, $R^7$, and $R^8$ are each independently selected from the group consisting of H, $^2H$, halogen, $C_{1-3}$alkyl, $C_{1-3}$alkoxy, $C_{3-7}$ cycloalkyl and 4-7 membered heterocyclyl having one, two or three heteroatoms each independently selected

from the group consisting of N, O and S; wherein $C_{1-3}$alkoxy may optionally be substituted by one, two, three or more halogens; and wherein $C_{1-3}$alkyl may optionally be substituted by one, two, three or more substituents, each independently selected from the group consisting of hydroxyl, halogen and $C_{1-3}$alkoxy (optionally substituted by one or more halogens); or

wherein $R^6$ and $R^8$ optionally forms a partially unsaturated carbobicyclic or heterobicyclic ring with the heteroaryl ring to which they are attached, wherein the carbobicyclic or heterobicyclic ring may optionally be substituted by one, two or three groups, each independently selected from the group consisting of $^2$H, halogen, $C_{1-3}$alkyl, $C_{1-3}$alkoxy, $C_{3-7}$cycloalkyl and a 4-7 membered heterocyclyl having one, two or three heteroatoms each independently selected from the group consisting of N, O and S; wherein $C_{1-3}$alkyl and $C_{1-3}$alkoxy may optionally be substituted by one, two, three or more halogens; or

tautomers, stereoisomers, or pharmaceutically acceptable salts thereof or esters thereof.

For example, contemplated PKC inhibitors may be represented by Formula II wherein X is CR; $R^2$, $R^3$ and $R^4$ are each H; $R^5$ is independently H, $CH_3$, $CH_2F$, $CHF_2$, $CF_3$, $CH_2OH$, and $CH_2$-O-$C_{1-3}$ alkyl; $R^{5a}$ and $R^{5b}$ are each H; $R^{5c}$ and $R^{5d}$ are each independently H, F, $C_{1-3}$ alkyl, or $C_{1-3}$ alkoxy or $R^{5c}$ and $R^{5d}$ are joined together forming a methylene, ethylene or -$CH_2$-O- bridging group; and $R^6$ and $R^7$ are each independently selected from H, halo, $C_{1-3}$ haloalkyl, $C_{1-3}$haloalkoxy, $C_{3-7}$ cycloalkyl, morpholino, piperinyl and piperazinyl.

For example, a contemplated PKC inhibitor may be represented by Formula III:

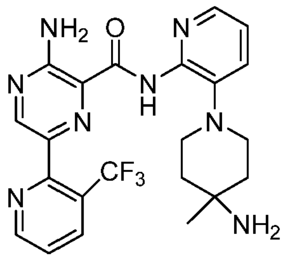

,

or a pharmaceutically acceptable salt thereof.

It can be appreciated that the disclosed methods may include administering the PKC inhibitor as part of a pharmaceutical compositions, for example, may include administering a PKC inhibitor compound, such as those disclosed herein, in a dosage unit form. A pharmaceutical composition should be formulated to be compatible with its intended route of administration, i.e. the oral route.

**Mutation Detection**

Determining whether a patient has a specific mutation, for example, a *GNAQ* or *GNA11* genetic mutation, can include collecting and/or analyzing a patient sample. Patient samples of interest include, for example, carcinoma tissue, cancer tissue, solid tumor tissue, tumor tissue, body tissue, blood, serum, plasma, or body fluid, for example, circulating blood containing tumor DNA, obtained from the patient. Patient samples used in a method described herein can also include tissue samples such as, but not limited to, gastrointestinal, mucosal, submucosal, intestinal, esophageal, ileal, rectal, cervical, colonic, epidermal, lung, thymus, pancreatic, stomach, rectal, cutaneous, subcutaneous, or lymphatic samples. Samples may also include cellular samples, for examples, cutaneous cell samples. The presence of a genetic mutation of interest in a sample from a patient may be determined using various assays. For example, in methods of the disclosure , a genetic mutation in *GNAQ, GNA11, BAP1, SF3B1, EIF1AX, CDKN2A, TERT, BRAF NRAS,* and/or another gene or DNA sequence may be determined by nucleotide analysis, for example, genetic sequencing, Southern blotting, FISH, high-throughput sequencing, phage display, shotgun sequencing, or PCR, for example, by RT-PCR. Sequencing methods described herein may employ the use of DNA primer sequences targeted to specific gene sequences, for example, a gene sequence of *GNAQ, GNA11, BAP1, SF3B1, EIF1AX, CDKN2A, TERT, BRAF, or NRAS.*

The presence of a genetic mutation in a patient, for example a genetic mutation in *GNAQ, GNA11, BAP1, SF3B1, EIF1AX, CDKN2A, TERT, BRAF,* or *NRAS* may be analyzed by analyzing gene products of a gene of interest, including mRNA and protein levels of the *GNAQ, GNA11, BAP1, SF3B1, EIF1AX, CDKN2A, TERT, BRAF,* or *NRAS* genes. For example, in some embodiments, the presence of one or more genetic mutations is analyzed by analyzing GNAQ, GNA11, BAP1, SF3B1, EIF1AX, CDKN2A, TERT, BRAF, or NRAS protein or mRNA. The presence of one or more genetic mutations in *GNAQ, GNA11, BAP1, SF3B1, EIF1AX, CDKN2A, TERT, BRAF,* or *NRAS* identified at the protein or mRNA

level may be determined using various detection methods. For example, in some embodiments, the presence of one or more genetic mutations in *GNAQ, GNA11, BAP1, SF3B1, EIF1AX, CDKN2A, TERT, BRAF,* or *NRAS* identified at the protein or mRNA level is determined by immunohistochemistry or by nucleotide analysis.

In addition, methods of the disclosure may also include a determining if a genetic mutation is associated with a gene other than *GNAQ, GNA11, BAP1, SF3B1, EIF1AX, CDKN2A, TERT, BRAF,* or *NRAS.*

Methods of determining the presence of one or more genetic mutations in *GNAQ, GNA11, BAP1, SF3B1, EIF1AX, CDKN2A, TERT, BRAF,* or *NRAS* include, but are not limited to, methods of analyzing analyte mRNA transcripts such as polymerase chain reaction methods, for example, quantitative polymerase chain reaction methods. Nucleotide analysis may be performed using an oligonucleotide probe that binds an analyte nucleotide sequence (*e.g.,* a GNAQ, GNA11, BAP1, SF3B1, EIF1AX, CDKN2A, TERT, BRAF, or NRAS nucleotide sequence) or a pair of oligonucleotide primers capable of amplifying an analyte nucleotide sequence via a polymerase chain reaction, for example, by a quantitative polymerase chain reaction. Oligonucleotide probes and oligonucleotide primers may be linked to a detectable tag, such as, for example, a fluorescent tag. In determining the presence of one or more genetic mutations in *GNAQ, GNA11, BAP1, SF3B1, EIF1AX, CDKN2A, TERT, BRAF,* or *NRAS* by nucleotide analysis, the practitioner may evaluate a particular gene's mRNA transcript makeup or concentration in a sample.

The presence of one or more genetic mutations in *GNAQ, GNA11, BAP1, SF3B1, EIF1AX, CDKN2A, TERT, BRAF,* or *NRAS* in a patient may be determined by obtaining a sample from the patient. For example, in some embodiments, the presence of one or more genetic mutations in *GNAQ, GNA11, BAP1, SF3B1, EIF1AX, CDKN2A, TERT, BRAF,* and/or *NRAS* in the patient is determined in a sample obtained from the patient. In some embodiments, the sample is a blood, serum, plasma, tumor, or tissue sample. According to the methods described herein, a sample may be a tissue sample (*e.g.,* an intestinal tissue sample, a duodenal tissue sample, a jejunal tissue sample, a stomach sample, a pancreatic tissue sample, a lung tissue sample, a gastrointestinal tissue sample, a rectal tissue sample, a colonic tissue sample, or a cervical tissue sample), a tumor sample (for example, a carcinoma sample, a solid tumor sample, or a bodily fluid sample that includes tumor DNA), or a bodily fluid sample (*e.g.,* a saliva sample, a stool, or a urine sample). A sample can be a sample obtained from a patient tissue biopsy, for example, a mucosal tissue biopsy, for example, an intestinal mucosal tissue biopsy, for example a small intestinal mucosal tissue biopsy. Furthermore, the sample may be a blood, serum, or plasma sample. A blood sample from a subject may be obtained using techniques well-known in the art. Blood samples may include peripheral blood mononuclear cells (PBMCs), RBC-depleted whole blood, or blood serum. PBMCs can be separated from whole blood samples using different density gradient (*e.g*., Ficoll density gradient) centrifugation procedures. For example, whole blood (*e.g.,* anticoagulated whole blood) is layered over the separating medium and centrifuged. At the end of the centrifugation step, the following layers are visually observed from top to bottom: plasma/platelets, PBMC, separating medium and erythrocytes/granulocytes.

Methods of the claimed disclosure include steps that may be carried out *in vitro*. For instance, it is contemplated that the steps of the presence of one or more genetic mutations in *GNAQ, GNA11, BAP1, SF3B1, EIF1AX, CDKN2A, TERT, BRAF,* and/or *NRAS* in the subject may involve determining the presence of one or more genetic mutations in *GNAQ, GNA11, BAP1, SF3B1, EIF1AX, CDKN2A, TERT, BRAF,* and/or *NRAS* in a sample. For example, the presence of one or more genetic mutations in *GNAQ, GNA11, BAP1, SF3B1, EIF1AX, CDKN2A, TERT, BRAF,* and/or *NRAS* in a sample may be determined by performing nucleotide analysis on the sample *in vitro.* Alternatively, in some embodiments of the disclosure, the steps of determining and analyzing the presence of one or more genetic mutations in *GNAQ, GNA11, BAP1, SF3B1, EIF1AX, CDKN2A, TERT, BRAF,* and/or *NRAS* in a patient may be carried out *in vivo.*

The methods described herein contemplate identifying the presence of one or more genetic mutations in *e.g., GNAQ, GNA11, BAP1, SF3B1, EIF1AX, CDKN2A, TERT, BRAF,* and/or *NRAS* by analyzing a patient or a sample from a patient, for example, a tumor, blood, or tissue sample from a patient. Analyzing a patient or a sample from a patient, for example, a tumor, blood, or tissue sample from a patient, can include collecting, purifying, and/or extracting DNA from the patient or the sample. DNA collection, purification, and extraction techniques are well-known to those skilled in the art. In general, DNA extraction requires collecting cells that contain DNA for analysis, and breaking down cell membranes to expose DNA. DNA extraction may entail steps of concentrated salt solution treatment, centrifugation to separate DNA from other cellular components, and DNA purification using, for example, ethanol precipitation, phenol-chloroform extraction, and/or minicolumn purification. Common DNA extraction methods include organic extraction, Chelex extraction, and solid phase extraction. DNA purification can include collecting a blood sample to analyze for the presence of circulating tumor cells or free DNA from tumor cells. Kits available for collecting cell-free tumor DNA include MagNA Pure Compact (MPC) Nucleic Acid Isolation Kit I, Maxwell® RSC (MR) ccfDNA Plasma Kit, and the QIAamp Circulating Nucleid Acid (QCNA) Kit.

The presence of one or more genetic mutations in *GNAQ, GNA11, BAP1, SF3B1, EIF1AX, CDKN2A, TERT, BRAF,* and/or *NRAS* can be accomplished for example, using any method known in the art. In general, presence is detected in a sample taken from the patient. Identification of the presence of a genetic mutation in a patient can be accomplished using standard techniques, including hybridization-based techniques, sequencing techniques, and array-based techniques.

The determination of the presence of one or more genetic mutations in *GNAQ, GNA11, BAP1, SF3B1, EIF1AX,*

*CDKN2A, TERT, BRAF,* and/or *NRAS* can be detected in any biological sample and can be any specimen obtained from a patient or test subject that contains a nucleic acid (*e.g.,* genomic DNA or RNA) that encodes *GNAQ, GNA11, BAP1, SF3B1, EIF1AX, CDKN2A, TERT, BRAF,* and/or *NRAS*. Exemplary samples include a tissue biopsy, cell, bodily fluid (*e.g.,* blood, serum, plasma, semen, urine, saliva, amniotic fluid, or cerebrospinal fluid).

Once obtained, the presence of one or more genetic mutations in *GNAQ, GNA11, BAP1, SF3B1, EIF1AX, CDKN2A, TERT, BRAF,* and/or *NRAS* can be detected using any appropriate method. In some embodiments, a hybridization approach is used. Hybridization approaches include dynamic allele-specific hybridization (Howell et al., Nat. Biotechnol. 17:87, 1999). This approach relies on differential melting temperatures between the sequence containing the polymorphism as compared to the sequence without the polymorphism. Briefly, a DNA region of interest is amplified by PCR using a biotinylated primer. The resulting PCR product is attached to a streptavidin support and is hybridized to an allele-specific probe in the presence of a DNA duplex-binding fluorescent molecule. The duplex is heated, and the temperature at which the duplex denatures is determined based on loss of fluorescence. The denaturation temperature is determinative of the presence or absence of the polymorphism or mutation.

Another hybridization approach for identifying the presence of SNPs is the use of nucleic acid arrays designed for this purpose. For example, arrays designed to detect one or more genetic mutations in *GNAQ, GNA11, BAP1, SF3B1, EIF1AX, CDKN2A, TERT, BRAF,* and/or *NRAS* can be used to detect the presence of genetic mutations in samples taken from a patient.

In some embodiments, presence of one or more genetic mutations in *GNAQ, GNA11, BAP1, SF3B1, EIF1AX, CDKN2A, TERT, BRAF,* or *NRAS* may be determined by performing a "nucleotide analysis." A nucleotide analysis may include analysis of analyte nucleotide transcript levels (*e.g., GNAQ, GNA11, BAP1, SF3B1, EIF1AX, CDKN2A, TERT, BRAF,* or *NRAS* mRNA transcript levels) in a sample, for example, a blood sample. Analyte transcript levels may be determined by Northern blot, for example, a quantitative Northern blot; or polymerase chain reaction, for example, a quantitative polymerase chain reaction. Reagents necessary to perform Northern blot include oligonucleotide probes, for example, oligonucleotide probes linked to a detectable label. A nucleotide analysis may include analysis to determine the gene and/or mRNA sequence of a gene of interest (*e.g., GNAQ, GNA11, BAP1, SF3B1, EIF1AX, CDKN2A, TERT, BRAF,* or *NRAS*). Methods of determining genetic sequence include PCR, RNA sequencing, RNA-seq, ChIP-sequencing, Massively parallel signature sequencing (MPSS), Nanopore DNA sequencing, Polony sequencing, 454 pyrosequencing (454 Life Sciences), Illumina (Solexa) sequencing, Single molecule real time (SMRT) sequencing (Pacific Biosciences), Combinatorial probe anchor synthesis (cPAS), SOLiD sequencing (Applied Biosystems), Ion Torrent semiconductor sequencing (Ion Torrent Sequencing; Life Technologies), DNA nanoball sequencing, Heliscope single molecule sequencing, phage display, *de novo* sequencing, bridge PCR, Southern blotting, shotgun DNA sequencing, and high throughput DNA sequencing. Detectable labels may include fluorescent labels or enzymes capable of reacting with a specific substrate. Reagents necessary to perform polymerase chain reaction include oligonucleotide primers capable of specifically binding to a particular analyte mRNA transcript and amplifying the number of analyte mRNA transcripts by polymerase chain reaction. Oligonucleotide primers may be linked to a detectable label to enable, for example, quantitative polymerase chain reaction. Other reagents necessary to perform quantitative polymerase chain reaction include, but are not limited to, primers capable of amplifying a control transcript signal, for instance, a beta tubulin transcript signal. Buffers, reagents (including oligonucleotide primers and probes), techniques, and equipment necessary for performing nucleotide sequencing methods described herein are readily available and are well-known in the art.

The methods described herein include, in some embodiments, determining if a cancer, tumor, or carcinoma has a *GNAQ* or *GNA11* genetic mutation and/or a *GNAQ, GNA11, BAP1, SF3B1, EIF1AX, CDKN2A, TERT, BRAF,* or *NRAS* genetic mutation, methods wherein the patient has been determined to have a *GNAQ* or *GNA11* genetic tumor mutation and/or a *GNAQ, GNA11, BAP1, SF3B1, EIF1AX, CDKN2A, TERT, BRAF,* or *NRAS* genetic mutation, methods of treating a cancer, for example, a solid tumor cancer having a *GNAQ* or *GNA11* genetic mutation and/or a *GNAQ, GNA11, BAP1, SF3B1, EIF1AX, CDKN2A, TERT, BRAF,* or *NRAS* genetic mutation in a patient, and identifying a patient as having a tumor with a mutation that includes a specific genetic mutation, for example, a substitution of arginine in codon R183 in *GNAQ* or *GNA11.* Genetic mutations can include any of the following: insertion mutations, substitution mutations, deletion mutations, gain of function mutations, loss of function mutations, and non-synonymous mutations. An insertion mutation is the addition of one or more nucleotide base pairs into a DNA sequence. A substitution mutation can be caused by chemicals or malfunction of DNA replication, and include the exchange of a single nucleotide for another. A deletion mutation removes one or more nucleotides from the DNA, and can alter the reading frame of the gene. A gain of function mutation results in an altered gene product that possesses a new molecular function or a new pattern of gene expression. A loss of function mutation produces an altered gene product that lacks the molecular function of the equivalent wild-type gene. A non-synonymous is a genetic mutation that alters the resulting amino acid encoded by the gene.

The disclosure now being generally described, it will be more readily understood by reference to the following examples which are included merely for purposes of illustration of certain aspects and embodiments of the present disclosure , and are not intended to limit the disclosure in any way.

## EXAMPLES

The following examples are merely illustrative and are not intended to limit the scope or content of the disclosure in any way.

### Example 1 - Identification of mutations in either *GNAQ* or *GNA11*

The frequency of all alterations on *GNAQ* and *GNA11* across tumor types was determined using three cancer /tumor based datasets: The Cancer Genome Atlas (TCGA), Genomics Evidence Neoplasia Information Exchange (Genie), and Memorial Sloan Kettering-Integrated Mutation Profiling of Actionable Cancer Targets (MSK-Impact). The number of tumor samples evaluated in each dataset included 9136 tumor samples from TCGA, 48451 tumor samples from Genie, and 10949 tumor samples from MSK-Impact. Many *GNAQ* and *GNA11* alterations were identified including the activating mutations at Q209 and R183 - gain of function mutations that confer dependence on downstream PKC signaling. In order to rank these mutations for downstream validation and to assess prevalence of amino acid recurrence which resulted from missense mutations across all samples, mutation type ratio analysis was performed. The analysis of synonymous vs non-synonymous ratios was performed using the TCGA and Genie datasets.

This ranking analysis led to the identification of 294 mutations other than mutations at Q209 or R183, in either *GNAQ* or *GNA11* and with a combined prevalence in tumor samples across all three datasets ranging between 1 to 8 total samples per mutation. While 139 mutations (other than Q209 or R183 mutations) were identified for *GNAQ,* a total of 155 non-Q209/R183 mutations were identified for *GNA11,* at least some of which may be activating mutations that confer dependence on downstream PKC signaling.

Following further analysis, 38 other, non-Q209/R183, mutations were identified in either *GNAQ* or *GNA11* with a prevalence of 3 or more samples across all three datasets. These other mutations show enrichment in colorectal, lung, endometrial, and bladder cancers along with melanoma and glioma. These mutations have been listed in Table 1. These results demonstrate the enrichment of other mutations in *GNAQ* or *GNA11* in certain non-uveal forms of cancer, *e.g.*, colorectal, non-small cell lung cancer, pancreatic cancer.

Table 1. Mutation analysis. [Abbreviations used in Table 1 - HUGO (Human Genome Organization); AA (Amino Acid)]

| HUGO_ Symbol | AA position | Reference AA | Total samples | Protein change | Cancer type |
|---|---|---|---|---|---|
| GNA11 | 256 | R256 | 8 | p.R256Q,p.R256W | TCGA-HNSC,Genie-Cancer of Unknown Primary, Genie-Endometrial Cancer, CCLE-NA, Genie-Glioma, Genie-Breast Cancer, Genie-Germ Cell Tumor,Genie-Colorectal Cancer |
| GNAQ | 279 | L279 | 8 | p.L279P | Genie-Colorectal Cancer,Genie-Melanoma,Genie-Non-Small Cell Lung Cancer |
| GNA11 | 166 | R166 | 6 | p.R166H | TCGA-HNSC,TCGA-PAAD,TCGA-UVM,Genie-Prostate Cancer,Genie-Colorectal Cancer, Genie-Breast Cancer |
| GNA11 | 168 | A168 | 5 | p.A168T | CCLE-NA,CCLE-haematopoietic_and_lymphoid_tissue,Genie-Endometrial Cancer, Genie-Colorectal Cancer |
| GNA11 | 210 | R210 | 5 | p.R210L,p.R210Q,p.R210W | Genie-Non-Small Cell Lung Cancer,CCLE-breast,CCLE-haematopoietic_and_lymphoid_tissue,Genie-Colorectal Cancer |
| GNA11 | 213 | R213 | 5 | p.R213Q,p.R213W | Genie-Pancreatic Cancer, Genie-Mesothelioma,TCGA-HNSC |
| GNAQ | 166 | R166 | 5 | p.R166C,p.R166H,p.R166L | Genie-Pancreatic Cancer,CCLE-haematopoietic_and_lymphoid_tissue,Genie-Colorectal Cancer, Genie-Endometrial Cancer, Genie-Bladder Cancer |
| GNAQ | 338 | R338 | 5 | p.R338C,p.R338H | Genie-Colorectal Cancer,Genie-Bladder Cancer, CCLE-endometrium, Genie-Salivary Gland Cancer, Genie-Endometrial Cancer |
| GNA11 | 231 | A231 | 4 | p.A231T,p.A231V | TCGA-PRAD,Genie-Endometrial Cancer, Genie-Melanoma |
| GNA11 | 342 | A342 | 4 | p.A342T,p.A342V | TCGA-PAAD,CCLE-thyroid,Genie-Ovarian Cancer,Genie-Colorectal Cancer |
| GNA11 | 333 | D333 | 4 | p.D333N,p.D333Y | TCGA-UCEC,Genie-Non-Small Cell Lung Cancer, Genie-Endometrial Cancer,Genie-Skin Cancer, Non-Melanoma |
| GNA11 | 171 | G171 | 4 | p.G171D,p.G171S | Genie-Glioma,Genie-Breast Cancer, TCGA-SKCM,Genie-Melanoma |
| GNA11 | 147 | R147 | 4 | p.R147C | CCLE-prostate,Genie-Non-Small Cell Lung Cancer,Genie-Colorectal Cancer |
| GNA11 | 73 | R73 | 4 | p.R73H | Genie-Skin Cancer, Non-Melanoma,Genie-Glioma, Genie-Colorectal Cancer, Genie-Small Bowel Cancer |
| GNA11 | 47 | T47 | 4 | p.T47K,p.T47M | Genie-Endometrial Cancer, Genie-Cancer of Unknown Primary,Genie-Colorectal Cancer |

(continued)

| HUGO_ Symbol | AA position | Reference AA | Total samples | Protein change | Cancer type |
|---|---|---|---|---|---|
| GNAQ | 191 | E191 | 4 | p.E191K,p.E191Q | Genie-Skin Cancer, Non-Melanoma,Genie-Colorectal Cancer, Genie-Ovarian Cancer, Genie-Myelodysplasia |
| GNAQ | 221 | E221 | 4 | p.E221G,p.E221K,p.E221Q | Genie-Glioma,Genie-Non-Small Cell Lung Cancer,TCGA-BLCA,Genie-Bladder Cancer |
| GNAQ | 149 | R149 | 4 | p.R149Q | Genie-Skin Cancer, Non-Melanoma,Genie-Prostate Cancer |
| GNAQ | 175 | T175 | 4 | p.T175M,p.T175S | TCGA-COAD,Genie-Colorectal Cancer |
| GNAQ | 329 | T329 | 4 | p.T329M,p.T329R | CCLE-large_intestine, Genie-Non-Small Cell Lung Cancer, Genie-Colorectal Cancer, Genie-Cancer of Unknown Primary |
| GNAQ | 85 | T85 | 4 | p.T85M,p.T85P | TCGA-COAD,Genie-Hepatobiliary Cancer, Genie-Bladder Cancer, TCGA-LIHC |
| GNA11 | 86 | A86 | 3 | p.A86T | TCGA-BLCA,Genie-Soft Tissue Sarcoma, Genie-Gastrointestinal Neuroendocrine Tumor |
| GNA11 | 163 | D163 | 3 | p.D163N | Genie-Endometrial Cancer, Genie-Colorectal Cancer,Genie-Pancreatic Cancer |
| GNA11 | 195 | D195 | 3 | p.D195N | Genie-Skin Cancer, Non-Melanoma,Genie-Colorectal Cancer,Genie-Non-Small Cell Lung Cancer |
| GNA11 | 319 | D319 | 3 | p.D319N | CCLE-breast,Genie-Glioma,Genie-Colorectal Cancer |
| GNA11 | 191 | E191 | 3 | p.E191K | TCGA-LGG,Genie-Endometrial Cancer, Genie-Glioma |
| GNA11 | 280 | E280 | 3 | p.E280K,p.E280Q | Genie-Skin Cancer, Non-Melanoma,Genie-Cancer of Unknown Primary,Genie-Breast Cancer |
| GNA11 | 49 | E49 | 3 | p.E49K | Genie-Colorectal Cancer |
| GNA11 | 293 | P293 | 3 | p.P293S | TCGA-SKCM,Genie-Skin Cancer, Non-Melanoma,Genie-Melanoma |
| GNA11 | 300 | R300 | 3 | p.R300Q,p.R300W | Genie-Bladder Cancer, Genie-Colorectal Cancer, CCLE-endometrium |
| GNA11 | 338 | R338 | 3 | p.R338C | CCLE-endometrium, Genie-Endometrial Cancer,Genie-Thyroid Cancer |
| GNA11 | 60 | R60 | 3 | p.R60C | CCLE-large_intestine,CCLE-NA,Genie-Glioma |
| GNAQ | 155 | D155 | 3 | p.D155H | Genie-Breast Cancer, Genie-Bladder Cancer |

(continued)

| HUGO_ Symbol | AA position | Reference AA | Total samples | Protein change | Cancer type |
|---|---|---|---|---|---|
| GNAQ | 205 | D205 | 3 | p.D205H,p.D205N | Genie-Esophagogastric Cancer, Genie-Non-Small Cell Lung Cancer,Genic-Bladder Cancer |
| GNAQ | 321 | D321 | 3 | p.D321E,p.D321N,p.D321Y | TCGA-LUAD,Genie-Breast Cancer,Genie-Glioma |
| GNAQ | 226 | 1226 | 3 | p.I226M,p.I226V | Genie-Bladder Cancer,TCGA-CESC |
| GNAQ | 37 | R37 | 3 | p.R37H | CCLE-large_intestine, Genie-Colorectal Cancer |
| GNAQ | 240 | V240 | 3 | p. V240L,p. V240M | Genie-Esophagogastric Cancer, Genie-LeukemiaGenie-Pancreatic Cancer |

An analysis was conducted using cBioPortal (Combined Studies including a total of 72175 samples). Table 2 shows the number of patients with a cancer type and mutation type (*e.g.,* GNAQ Q209 mutation or a non Q209 mutation).

Table 2: cBioPortal analysis summary. [Abbreviations used in Table 2: n = number of samples associated with the cancer type; HCC = hepatocellular carcinoma; GBM = glioblastoma]

| Cancer Type/Mutation | GNAQ-Q209 | GNA11-Q209 | GNAQ-Non Q209 | GNA11-Non Q209 | n | GNAQ/GNA11 |
|---|---|---|---|---|---|---|
| Cutaneous Melanoma | 0.6% | 1% | 1.8% | 1.5% | 1510 | 4.9% |
| Colorectal | 0.1% | 0.05% | 1% | 2% | 3799 | 3.2% |
| Uterine | 0% | 0% | 1.8% | 1.3% | 1418 | 3.1% |
| Stomach | 0% | 0% | 0.8% | 1.5% | 1583 | 2.3% |
| Cervical | 0% | 0% | 0.7% | 1% | 297 | 1.7% |
| Bladder | 0% | 0% | 1% | 0.3% | 2215 | 1.3% |
| Lung Adeno | 0.02% | 0.04% | 0.6% | 0.4% | 4953 | 1.06% |
| HCC | 0% | 0% | 1% | 0% | 1165 | 1% |
| Pancreatic | 0.14% | 0% | 0.14% | 0.21% | 1409 | 0.5% |
| Prostate | 0% | 0% | 0.2% | 0.3% | 4932 | 0.5% |
| Breast | 0% | 0% | 0% | 0.5% | 4781 | 0.5% |
| Head & Neck | 0% | 0% | 0% | 0.4% | 1530 | 0.4% |
| GBM | 0% | 0% | 0% | 0.2% | 2273 | 0.2% |

**Example 2 - Structural analysis of mutations in either *GNAQ* or *GNA11***

Three-dimensional coordinates were downloaded for murine Galpha-q (R183C) protein in complex with G protein signaling 2 (RGS2) from the RCSB protein data bank. The protein was prepared by adding hydrogens and adjusting the protonation states of ionizable groups using the protein preparation tool and epik within Maestro 2018-3. This was followed by a restrained minimization to converge the heavy atoms to an RMSD of 0.3 Å. Activating mutations that confer dependence on downstream PKC signaling were mapped onto the protein and visual inspection revealed residues in contact with the GDP binding site as well as secondary residues directly interacting with structural motifs involved in binding to GTP. A homology model of human GNA11 (GenBank: CAG33285.1) was built using a crystal structure of human regulator of G protein signaling 2 (RGS2) in complex with murine Galpha-q(R183C) (4EKC) as the template protein. Prime 5.3 was employed to build a single template, single chain model with the ligand GDP from 4EKD. Sidechain positions were predicted for non-conserved residues by mutating the template protein residue to the desired identity. Subsequently, the first low energy rotomer to not produce a clash was retained. This was followed by optimization of all non-template atoms using a Ca-Cb bond angle sampling and minimization using OPLS3e forcefield in a VSGB solvation model for all residues. A restrained minimization using a Broyden-Fletcher-Goldfarb-Shanno method to converge the heavy atoms to an RMSD (root-mean-square deviation, and a measure to assess the structural similarity between two macromolecules)of 0.3 Å. Activating mutations that confer dependence on downstream PKC signaling were mapped onto the protein as shown in **FIGURE 1** and **FIGURE 2** and visual inspection revealed residues in contact with the GDP binding site as well as secondary residues directly interacting with structural motifs involved in binding to GTP. This data indicates that mutations (including mutations other than Q209 or R183), associated with *GNAQ* and *GNA11* in non-uveal forms of cancer affect G protein function.

**Example 3 - A Study of Protein kinase C inhibitor (IDE196) for the Treatment of Patients with Solid Tumors Harboring *GNAQ/11* Mutations**

This example describes the phase 1/2 study of compound of Formula (III) above, ("IDE196"), in patients with solid tumors harboring *GNAQ/11* mutations. The study consists of two phases: a dose escalation phase, followed by a dose expansion phase.

1. *Objectives of the Study*

**I. Primary Objectives:** Following are primary objectives of this study:

- Evaluation of safety profile of IDE196 in enrolled patients (Phase 1 dose escalation);
- Identification of maximum tolerated dose (MTD) and/or recommended phase 2 dose (RP2D) (Phase 1 dose escalation);
- Characterization of pharmacokinetic (PK) profile of IDE196 administered as powder-in-capsules (PIC) or tablet (Phase 1 dose escalation)

**II. Secondary Objectives:** Following are secondary objectives of this study:

- Evaluation of anti-tumor activity of IDE196 in all patients;
- Evaluation of anti-tumor activity of IDE196 in non-metastatic uveal melanoma (non-MUM) patients as determined by Investigator (INV);
- Evaluation of comprehensive safety and tolerability of IDE196 for all patients;
- Assessment of pharmacodynamic (PD) effect of IDE196 in all patients.

**III. Exploratory Objectives:** Following are exploratory objectives of this study:

- Characterization of preliminary anti-tumor activity of IDE196 in all patients;
- Exploration of any correlation of tumor genetic and molecular profiles and response to IDE196 in all patients;
- Exploration of any association of PD effect in DNA derived from blood or tumors with clinical response.

2. *Endpoints of the Study*

**I. Primary Endpoints:** Following are primary endpoints of this study:

- Incidence of grade 3 or 4 adverse events (AEs) and clinically significant laboratory abnormalities defined as dose-limiting toxicities (DLTs) (also discussed later in *Section 4*);
- PK parameters:
    - ◦ Area under the curve (AUC) from time zero to time t ($AUC_{0-t}$), defined as the AUC calculated from 0 to specified time points (amount x time x $volume^{-1}$), for example, 8, 24, 48, 168 hours or infinity.
    - ◦ AUC from time zero to infinity ($AUC_{0-\infty}$ or $AUC_{inf}$), defined as the area under the concentration-time curve from 0 to infinity [mass x time x $volume^{-1}$].
    - ◦ AUC over the dosing interval ($AUC_{tau}$), defined as area under the concentration-time curve from time 0 to the end of the dosing interval tau.
    - ◦ Maximum concentration ($C_{max}$), defined as maximum observed plasma concentration after drug administration [mass x $volume^{-1}$].
    - ◦ Time to maximum concentration ($T_{max}$), defined as time to reach $C_{max}$ [time].
    - ◦ Elimination half-life (T½), defined as elimination half-life associated with the terminal slope (lambda z) of a semi-logarithmic concentration-time curve [time].
    - ◦ Apparent volume of distribution at steady state after administration ($V_{ss}/F$).
    - ◦ Apparent total plasma clearance (CL/F).
- Blinded independent central review (BICR)- determined objective response rate (ORR) per Response Evaluation Criteria in Solid Tumors (RECIST) Version 1.1 (v1.1).

**II. Secondary Endpoints:** Following are secondary endpoints of this study:

- BICR and INV- determined objective response rate (ORR) per RECIST v1.1;
- BICR-determined duration of response (DOR) per RECIST v1.1;
- INV-determined ORR, DOR per RECIST v1.1;

- BICR and INV-determined disease control rate lasting ≥ 6 months (DCR6) per RECIST v1.1;
- Incidence of AEs and clinically significant laboratory abnormalities;
- Modulation of signaling proteins in the PKC pathway, e.g., PKC-delta.

**III. Exploratory Endpoints:** Following are exploratory endpoints of this study:

- Progression-free survival (PFS) per RECIST v1.1 and overall survival (OS);

- Exploratory assessment of gene signatures and/or molecular profiling and ORR per RECIST v1.1;

- Exploratory assessment of treatment-related changes in tumor tissue or cell-free DNA from blood and ORR per RECIST v1.1.

*3. Study Design*

This is a phase 1/2, multi-center, open-label, dose-escalation, and expansion study of IDE196 in patients with solid tumors harboring *GNAQ*/*11* mutations. A schematic diagram of the study is described in FIGURE 3.

**I. Phase I Dose Escalation:** A standard 3+3 methodology is used to commence the IDE196 dose escalation portion of the study. For dose escalation, there are estimated three to five cohorts, with a minimum of three patients with metastatic uveal melanoma (MUM), and up to 6, enrolled in each cohort. Once a dose level has satisfied the DLT observation criteria (discussed in *Section 4*), patients with non-MUM tumors harboring *GNAQ*/*11* hotspot mutations are enrolled at that dose level, contributing to the safety and efficacy evaluations, but not to the DLT assessment. Each treatment cycle comprises continuous dosing over 28 days. All dose escalations are based on assessment of DLTs, overall safety and tolerability, and PK that occur with each cohort, and are agreed upon between the investigators and sponsor. Dose escalation may proceed if 0 of 3 or at most 1 of 6 patients experience a DLT during the DLT assessment period in Cycle 1 Days 1 - 28.

**Recommended phase 2 dose (RP2D)** may be selected based on one or more of the following: (1) the maximum tolerated dose (MTD), defined as the maximum daily oral dose at which no more than 1 in 6 patients (<33%) experience a DLT during Cycle 1 (safety and PK assessment period). If a DLT is observed in one of three patients, then three additional patients are enrolled at that same dose level. Dose escalation are continued until two or more of the three to six patients treated at a dose level experience a DLT. The next lower dose is then considered the MTD; (2) maximum administered dose (MAD), if no MTD is identified; (3) anti-tumor, PK and/or PD results; and/or (4) the occurrence, nature and severity of toxicities occurring after Cycle 1.

**II. Phase 2 Dose Expansion:** Once RP2D is established,

A. *GNAQ*/*11*-hotspot mutated tumor cohorts: IDE196 treatment has demonstrated preliminary anti-tumor activity in patients with MUM. Up to 24 patients each with a) cutaneous melanoma or b) colorectal cancer (CRC) harboring *GNAQ*/*11* hotspot mutations are enrolled. Mutations are defined as "hotspot" based on their significantly higher frequency of occurrence compared to background and does not indicate whether the gene product is activating. Patients with cutaneous melanoma or CRC tumors harboring mutations in codons Q209 or R183 of GNAQ or GNA11 as determined by local testing in a College of American Pathologists (CAP)/certified through Clinical Laboratory Improvement Amendments (CLIA) certified laboratory, are enrolled. Independent central testing is conducted retrospectively to confirm *GNAQ*/*11* mutations on tumor and blood samples.

B. Other *GNAQ*/*11-hotspot* mutated tumors: Patients with other solid tumors (excluding MUM, cutaneous melanoma, and CRC) harboring *GNAQ*/*11* hotspot mutations in codons Q209 or R183 of GNAQ or GNA11 as determined by local testing in a CAP/CLIA certified laboratory, are enrolled. Independent central testing is conducted retrospectively to confirm *GNAQ*/*11* mutation on tumor and blood samples. Up to 10 eligible patients are enrolled in this cohort in total.

*4. Dose-Limiting Toxicities (DLT)*

A dose-limiting toxicity (DLT) is defined as a drug-related (or at least possibly related) event grade 3 or higher adverse event or abnormal laboratory value assessed as unrelated to disease, disease progression, inter-current illness, or concomitant medications that occurs within the first cycle of study drug with IDE196 and meets any of the criteria included below:

**Hematology:**

- ≥ grade 4 neutropenia for > 7 consecutive days;
- ≥ grade 3 febrile neutropenia (defined as absolute neutrophil count [ANC] < 1000 $mm^3$, with a single temperature of 38.3 °C (101 °F) or a sustained temperature of 38.0 °C (100.4 °F) for > 1 hour;
- grade 4 thrombocytopenia;
- grade 3 thrombocytopenia associated with ≥ grade 2 bleeding.

**Hepatic:**

For patients with normal baseline aspartate aminotransferase (AST) and alanine aminotransferase (ALT) and total bilirubin values:

◦ AST or ALT > 3 x upper limit of normal (ULN), combined with a total bilirubin > 2 x ULN and without evidence of cholestasis (by ALP < 2 x ULN), and no other cause identified.

- For patients with abnormal baseline AST and ALT and total bilirubin values:

  ◦ [AST or ALT > 2 x baseline AND > 3 x ULN] OR [AST or ALT > 8 x ULN], whichever is lower, combined with [total bilirubin > 2 x baseline AND > 2 x ULN].

- ≥ grade 3 direct bilirubin (> 3 x ULN);
- ≥ grade 2 direct bilirubin and ≥ Common Terminology Criteria for Adverse Events (CTCAE) grade 2 ALT.

**Other non-Hematologic Toxicities:**

- any ≥ grade 4 non-hematologic toxicity;
- any ≥ grade 3 non-hematologic toxicity lasting > 3 days despite optimal supportive care, except for exclusions listed under **Exceptions to DLT criteria;**
- any ≥ grade 3 non-hematologic laboratory value that is symptomatic and has persisted for > 3 days, except for exclusions listed under **Exceptions to DLT criteria.**

**Exceptions to DLT criteria:**

- CTCAE grade 3 fatigue lasting ≤ 7 days;
- CTCAE grade 3 laboratory abnormalities that are responsive to oral supplementation within 7 days or deemed to be clinically insignificant by the Investigator;
- CTCAE grade 3 or 4 lymphopenia unless judged to be clinically significant;
- Leukopenia in the absence of dose-limiting neutropenia or lymphopenia.

*5. Study Population:*

**I. Criteria for Inclusion-** Patient must meet all of the following inclusion criteria:

1. Patient must be at least 18 years of age.
2. Patient is able to provide written, informed consent before initiation of any study related procedures, and is able, in the opinion of the investigator, to comply with all the requirements of the study.
3. Patient population: The study is conducted in patients with solid tumors harboring *GNAQ*/*11* mutations, including, but not limited to, cutaneous melanoma and colorectal cancer. Patients with other solid tumors harboring the mutations of interest must have received prior standard therapies before being considered for this study.
4. Diagnosis of :

- Non-MUM: Advanced cutaneous melanoma, CRC, or other solid tumor that has either progressed following prior standard therapies or that has no satisfactory alternative therapies and has evidence of *GNAQ*/*11* hotspot mutation (codons Q209 or R183) by local testing in a CAP/CLIA-certified laboratory.

Representative archival metastatic tumor specimens in paraffin blocks with an associated pathology report or a minimum of 15 formalin-fixed paraffin-embedded FFPE slides is mandatory.
Only tissue from a surgical resection or a core needle, punch, or excisional/incisional biopsy sample collection is accepted. Fine needle aspiration (FNA) samples are not acceptable.

5. Patients with a prior history of or clinically stable concurrent malignancy are eligible for enrollment provided the malignancy is clinically insignificant, no treatment is required, and the patient is clinically stable.

- Patients with a history of squamous or basal cell carcinoma of the skin or carcinoma in the situ of the cervix may be enrolled.
- Patients with prostate cancer with an elevated PSA not requiring treatment may be enrolled.

6. Measurable disease per RECIST v1.1, defined as at least one lesion that can be accurately measured in at least one dimension (longest diameter to be recorded) as ≥ 10 mm with CT or MRI scan, or by digital photography with calipers and ruler for cutaneous lesions. An enlarged lymph node must be ≥ 15 mm in short axis to be a measurable lesion.

- Lesions in previously irradiated areas should not be considered target lesions unless they have clearly progressed since the radiotherapy.
- Liver lesions that received liver-directed therapies should not be considered target lesions unless they have clearly progressed since the therapy.
- For patients with metastases in the liver, these patients should have contrast-enhanced liver imaging modality preference determined by expertise at the treating institution.

7. Patient has Eastern Cooperative Oncology Group (ECOG) performance status 0 - 1.
8. Patient has adequate organ function at screening:

- Absolute neutrophil count ≥ 1500/$mm^3$ without the use of hematopoietic growth factors;
- Platelet count ≥ 75,000/$mm^3$ (must be at least 2 weeks post-platelet transfusion and not receiving platelet-stimulating agents);
- Hemoglobin ≥ 8.0 g/dL (must be at least 2 weeks post-red blood cell transfusion and not receiving erythropoietic-stimulating agents);
- Total bilirubin ≤ 1.5 x the upper limit of normal (ULN). For patients with documented Gilbert's disease, total bilirubin ≤ 3.0 mg/dL is allowed;
- ALT and AST ≤ 3 x ULN in the absence of documented liver metastases; ≤ 5 x ULN in the presence of liver metastases;
- Serum albumin ≥ 2.0 g/dL;
- Creatinine clearance ≥ 60 mL/min/1.73 $m^2$ by Cockroft-Gault equation[Creatine clearance = [(140-age) x weight in kg)] / (serum creatinine x 72)];
- Prothrombin time/International Normalized Ratio (INR) or partial thromboplastin time test results at screening ≤ 1.5 x ULN (this applies only to patients who do not receive therapeutic anticoagulation; patients receiving therapeutic anticoagulation should be on a stable dose for at least 4 weeks prior to the first dose of study drug).

9. Patients who received prior immune-stimulatory antitumor agents, such as anti-PD-1, anti-PD-L1, anti-CTLA-4, OX-40, CD137, etc., or MAPK pathway inhibitors may be eligible. Prior to study Day 1 (first dose), patient must be:

- at least 4 weeks or 5 half-lives (T½) after the most recent biologic (antibody-based) or immunotherapy, whichever is shorter;
- at least 2 weeks or 5 T½ after any prior systemic chemotherapy (> 6 weeks from nitrosourea and mitomycin C) or targeted small molecule therapy, whichever is shorter.

10. Female patients of childbearing potential must be non-pregnant, non-lactating, and have a negative serum

human chorionic gonadotropin pregnancy test result within 28 days prior to the first study drug administration.
11. Females of childbearing potential who are sexually active with a non-sterilized male partner agree to use effective methods of contraception from screening, throughout the study drug and agree to continue using such precautions for 30 days after the final dose of study drug.
12. Non-sterilized males who are sexually active with a female of childbearing potential must agree to use effective methods of contraception from Day 1 throughout the study drug and for 30 days after the final dose of study drug.
13. Patients must have exhausted all standard treatments or have documented intolerance per the investigator.
14. Archival metastatic tumor specimens in paraffin blocks with an associated pathology report or a minimum of 15 FFPE slides is mandatory. Only tissue from a surgical resection or a core needle, punch, or excisional/incisional biopsy sample collection will be accepted. Fine needle aspiration (FNA) samples are not acceptable.
15. Cutaneous melanoma:

- Histologically confirmed locally advanced and unresectable or metastatic melanoma with color medical grade photographs with a ruler if skin lesions present.
- Documented *RAF*/*RAS* wild-type status.

16. Colorectal cancer:

- Histologically confirmed locally advanced and unresectable or metastatic adenocarcinoma originating from the colon or the rectum.
- Documented RAF/RAS wild-type status and microsatellite stable (MSS) status

17. Phase 2: Hotspot mutations in other tumors:

- Patients must have exhausted all standard treatments or have documented intolerance per the investigator
- Patients must have the appropriate biomarker characterization for their tumor type *e.g.,* CA125, PSA, CEA

**Additional Inclusion Criteria for Biopsy-eligible non-MUM patients**

1. Accessible lesion(s) that permit a total of at least two biopsies (pretreatment and on-treatment) without unacceptable risk of a significant procedural complication. Acceptable samples include core needle biopsies for deep tumor tissue or lymph nodes or excisional, incisional, punch, or forceps biopsies for cutaneous, subcutaneous, or mucosal lesions. Fine needle aspirates, cell pellets from effusions or ascites, lavage samples, and bone biopsies are not permitted.

- Target lesions considered for core needle biopsies should be deemed suitable for retrieval of a minimum of three, but ideally five, cores at a given time point (minimum diameter 18-gauge).
- Alternatively, a minimum of two cores at a given time point with a 14-gauge needle is acceptable.
- If multiple lesions are available, it is preferable to obtain the on-treatment biopsy from the same lesion (or organ) as the pretreatment biopsy, if feasible.
- Biopsy from the same lesion (or organ) as the pretreatment biopsy, if feasible.

**II. General Exclusion Criteria for Phase I and Phase 2:** The presence of any of the following would exclude a patient from being eligible for the study:

1. Previous treatment with a PKC inhibitor.
2. Known microsatellite instability-high (MSI-H) tumors are excluded.
3. Have AEs from prior anti-cancer therapy that have not resolved to Grade ≤1 except for alopecia, prior peripheral neuropathy, or anemia. Endocrinopathies resulting from previous immunotherapy are considered part of the medical history and not an AE.
4. Untreated or symptomatic malignant lesions in the central nervous system (CNS). Patients with asymptomatic CNS lesions are eligible provided they have been clinically stable and not requiring steroids for at least 4 weeks.
5. Known human immunodeficiency virus (HIV) or acquired immunodeficiency syndrome (AIDS)-related illness.
6. Active infection requiring therapy (except nail fungus), positive tests for Hepatitis B surface antigen (HBsAg) with detected Hepatitis B virus (HBV) DNA or positive Hepatitis C antibody with detected Hepatitis C virus (HCV) RNA.

7. Surgical procedures that require general anesthesia ≤ 5 days prior to first scheduled dose of IDE196; in all cases, the patient must be sufficiently recovered and stable before study drug administration.
8. Prior gastrectomy or upper bowel removal or any other gastrointestinal disorder or defect *e.g.,* malabsorption disorder such as Crohn's disease or ulcerative colitis, that would interfere with absorption of IDE196.
9. Patients who received radiotherapy within 2 weeks of the first dose of study drug.
10. Patients who are receiving treatment with medications that cannot be discontinued prior to study entry and that are considered to be any of the following:

- known and possible risk for QT prolongation

11. Females who are pregnant or breastfeeding.
12. Women of childbearing potential must not be considering getting pregnant during the study.
13. Patients of reproductive potential (male and female) must practice an effective method of contraception during treatment and for 30 days following the last dose of IDE196. Patients unwilling to do so are excluded.
14. Impaired cardiac function or clinically significant cardiac diseases, including any of the following:

- History or presence of ventricular tachyarrhythmia.
- Presence of unstable atrial fibrillation (ventricular response > 100 BPM); patients with stable atrial fibrillation are eligible, provided they do not meet any of the other cardiac exclusion criteria.
- Angina pectoris or acute myocardial infarction ≤ 3 months prior to starting study drug.
- Other clinically significant heart disease (*e.g.,* symptomatic congestive heart failure; uncontrolled arrhythmia or hypertension; history of labile hypertension or poor compliance with an antihypertensive regimen).
- Patients with a drug eluting stent for cardiovascular purposes placed ≤ 2 months prior to starting study drug.
- Corrected QT interval using Fridericia's method (QTcF) > 480 msec on baseline ECG (mean of baseline values). If electrolytes are abnormal, they may be corrected and baseline ECGs should be repeated.
- **QT correction formula Fridericia** (*see* Luo S, Michler K, Johnston P, et al. (2004) A comparison of commonly used QT correction formulae: the effect of heart rate on the QTc of normal ECGs. Journal of Electrocardiology. 37:81-90)

$$QTcF = QT \div \sqrt[3]{RR}$$

15. Allergy to mammalian meat products or gelatin (for patients receiving IDE196 PIC only).
16. Presence of any other condition that may increase the risk associated with study participation or may interfere with the interpretation of study results and, in the opinion of the investigator, would make the patient inappropriate for entry into the study.

No waivers of inclusion or exclusion criteria are granted by the investigator and the sponsor or its designee for any patient enrolled into the study.

*6. Study Treatment*

*Dosage:* Each dose of IDE196 is ingested on a twice daily basis.

The starting dose in dose escalation is 300 mg twice daily (BID), which has been deemed safe and tolerable in an ongoing Phase I study CLXS196X2010 (EudraCT 2015-002158-11, NCT02601378). In certain exemplary embodiments, the subsequent planned dose levels are 400 mg BID. In certain exemplary embodiments, subsequent planned dose levels are 500 mg BID. There are no intra-patient dose escalation in the dose escalation cohorts.

*7. Concomitant Medications*

Concomitant antineoplastic therapies or investigational therapies other than IDE196 are prohibited. In addition, the following classes of medications are prohibited:

- Drugs with a known and possible risk of QT prolongation;
- Strong inhibitors and inducers of CYP3A4/5;
- Inhibitors and inducers of ABCB1 (P-gp);
- Substrates of CYP3A4/5 and /or ABCB1 with a Narrow Therapeutic Index (NTI);

- Substrates of OAT3, OATPIB1, and MATE1/2-K with a NTI.

The use of any concomitant non-cancer medication/therapy, including over-the-counter medications deemed necessary for the care of the patient or to treat AEs is permitted except for those specified as prohibited.

*8. Criteria for Withdrawal*

Study drug may be discontinued due to any of the following: adverse event, lost to follow-up, physician's decision, progressive disease, study terminated by the Sponsor, patient/guardian decision, protocol deviation, or non-compliance with the protocol. Study drug is discontinued if death or pregnancy occurs.

*9. Assessments*

**I. Pharmacokinetic Assessments:** PK sampling is taken at various timepoints after continuous twice daily dosing. For Phase I patients, on Cycle 1 Day 1 and Cycle 1 Day 15, the dose is followed by frequent PK sampling for 12 hours to determine the PK properties of IDE196 per the Schedule of Events. For Cycles 2 and beyond, only single trough-level PK samples are collected during each treatment cycle.

PK evaluation is based on the determination of the following parameters for IDE196 including, but not limited to: $AUC_{0-t}$, AUCO-∞, AUCtau, Cmax, Tmax, T½, Vss/F, CL/F. PK assay is conducted in a central laboratory.

**II. Pharmacodynamic Assessments:** Assays are conducted in a central laboratory.

**III. Efficacy Assessments:** Efficacy measures include tumor assessments, consisting of clinical examination and digital photography with calipers and rulers for cutaneous lesions, and appropriate imaging techniques (preferably computed tomography (CT) scans of the chest, abdomen, and pelvis with appropriate slice thickness per RECIST v1.1); other studies (magnetic resonance imaging [MRI], X-ray, positron emission tomography [PET], and ultrasound) may be performed if required.

Patients are evaluated for disease response per RECIST v1.1 with the first assessment being performed at screening, and then every 8 weeks (± 7 days) starting from the first dose for the first 12 cycles and every 12 weeks (± 7 days) thereafter.

**IV. Safety Assessments:** Safety is assessed through AEs, changes in laboratory tests, and changes in vital signs. The incidence and duration of toxicities per the NCI-CTCAE v5.0 are recorded. AEs are coded using the Medical Dictionary for Regulatory Activities (MedDRA) dictionary.

All treatment-emergent AEs are summarized as follows:

- Dose limiting toxicities;
- All AEs;
- All specific safety event categories (SEC) AEs;
- All Grade 3/4/5 AEs;
- All treatment-related AEs;
- All AES leading to study drug modifications or study discontinuations; and
- All SAEs, including deaths.

All AE, SAE, and DLT data is listed. Change from baseline for selected laboratory parameters is summarized by treatment group and scheduled visits. All laboratory test data are listed with abnormal values flagged.

Change from baseline for vital sign parameters is summarized by group and scheduled visits; all vital signs data are listed.

The MTD and RP2D for IDE196 is determined as a function of observed toxicity. PK and PD analyses and other parameters may be considered with determining RP2D.

*Tumor Tissue Samples*

Archival metastatic tumor tissue samples is mandatory.

**Expansion cohorts:** a minimum of 15 paired fresh tumor biopsies in non-MUM patients enrolled in the expansion cohorts are required at both screening and between Cycle 1 Day 15 - Cycle 2 Day 1. Tumor tissue biopsies at time of disease progression/discontinuation are optional for all patients.

*Statistical Procedures*

**For the non-MUM expansion cohorts,** the Simon 2-stage design is used to explore preliminary efficacy and to further characterize the safety profile of IDE196 therapy at the recommended phase 2 dose (R2PD).

**For each of the non-MUM expansion *GNAQ/11* hotspot mutation cohorts,** an ORR of 5% is of no clinical interest (null) whereas ORR of 20% is of interest. With a 10% type I error and 80% power, 9 patients are accrued in the first stage for each cohort. If zero responses are observed in the 9 patients for a cohort, no additional patients are enrolled to that cohort. Otherwise, 15 additional patients are accrued for a total of 24 in that cohort. Three or more responders are required from the 24 patients in a cohort to warrant further investigation.

**Exploratory cohorts** cover other *GNAQ*/*11*-hotspot mutated tumors. Approximately 10 patients with other GNAQ/11-hotspot mutated tumors are enrolled to assess ease of identification of these tumors and explore preliminary activity of IDE196 in this setting.

Analyses of safety, efficacy, PK, and PD is conducted. The analysis populations include:

- All enrolled population;
- Safety population;
- DLT evaluable population;
- Efficacy evaluable population; and
- Pharmacokinetic analysis population

Summaries of patient demographics, concomitant therapies, compliance is prepared and listed. Safety analyses include, but are not limited to, study drug administration, AEs, laboratory abnormalities, ECG evaluations, and vital signs. Efficacy analyses include preliminary anti-tumor activity as assessed by INV and BICR such as ORR, DOR, PFS, DCR, and OS. PK will be characterized in dose escalation patients at different doses of IDE196. The evaluation of PD effects will be based on PD markers such as PKC-delta and other exploratory pharmacodynamic biomarkers.

## Claims

1. A protein kinase C inhibitor for use in a method of treating a carcinoma having a *GNAQ* or *GNA11* genetic mutation in a patient in need thereof, wherein the carcinoma is selected from the group consisting of pancreatic adenocarcinoma, stomach adenocarcinoma, cervical carcinoma, and lung adenocarcinoma, the method comprising:

   determining if the carcinoma has a *GNAQ* or *GNA11* genetic mutation; and
   orally administering to the patient having the carcinoma with the *GNAQ* or *GNA11* genetic mutation a therapeutically effective amount of the protein kinase C inhibitor.

2. The protein kinase C inhibitor for use according to claim 1, wherein the *GNAQ* or *GNA11* mutation is a substitution, insertion and/or deletion mutation and/or a gain of function mutation.

3. The protein kinase C inhibitor for use according to claim 1 or 2, wherein:

   a. the *GNAQ* or *GNA11* genetic mutation is the substitution of glutamine in codon 209 (Q209); and/or
   b. the carcinoma has a *GNAQ* genetic mutation selected from Q209P, Q209L, Q209H, Q209K, and Q209Y, or a *GNA11* genetic mutation selected from Q209P, Q209L, Q209K, and Q209H; and/or
   c. the *GNAQ* or *GNA11* genetic mutation is Q209L.

4. The protein kinase C inhibitor for use according to claim 1 or 2, wherein:

   a. the *GNAQ* or *GNA11* genetic mutation is the substitution of arginine in codon 183 (R183), optionally wherein the *GNAQ* genetic mutation is R183Q; or
   b. the *GNAQ* or *GNA11* genetic mutation is other than a substitution of glutamine in codon 209 (Q209) and a substitution of arginine in codon 183 (R183), optionally wherein the *GNAQ* or *GNA11* genetic mutation activates the PKC pathway.

5. The protein kinase C inhibitor for use according to any one of claims 1-4, wherein:

   a. determining if the carcinoma has a *GNAQ* or *GNA11* genetic mutation comprises identifying the *GNAQ* or

*GNA11* genetic mutation in DNA extracted from a tumor sample or in circulating tumor DNA; and/or
b. determining comprises identifying the presence of a fusion gene in DNA extracted from a circulating tumor cell; and/or
c. the patient has a low tumor mutational burden of less than 17 mut/Mb, optionally wherein the patient has a low tumor mutational burden of less than 10 mut/Mb; and/or
d. the patient has a low tumor mutational load of mutations across the rest of the genome; and/or
e. the patient does not have activating BRAF or NRAS mutations; and/or
f. the patient has not been responsive to treatment with immune checkpoint inhibitors.

**6.** The protein kinase C inhibitor for use according to any one of claims 1-5, wherein:

a. administering comprises administering the protein kinase C inhibitor as monotherapy; or
b. administering further comprises administering at least one additional therapeutic agent; or
c. the method further comprises administering a therapeutically effective amount of a therapeutic agent selected from the group consisting of a MEK inhibitor, a mTOR inhibitor, a $CDK_i$ inhibitor, a HDM2-p53 inhibitor, an immune checkpoint inhibitor, and a combination of two or more thereof, optionally wherein the MEK inhibitor is selected from the group consisting of trametinib, cobimetinib, binimetinib, and selumetinib.

**7.** The protein kinase C inhibitor for use according to any one of claims 1-6, wherein:

a. the protein kinase C inhibitor has potency against one or more of $\delta$, $\varepsilon$, $\eta$, $\theta$, $\alpha$ protein kinase C isoforms; and/or
b. the protein kinase C inhibitor has potency against multiple protein kinase C isoforms; and/or
c. the protein kinase C inhibitor is a small molecule protein kinase C inhibitor; and/or
d. the protein kinase C inhibitor has an $IC_{50}$ with respect to PKC $\theta/\alpha$ and/or PKC $\delta$ and/or PKC $\varepsilon$ isoforms of less than 50 nM.

**8.** A protein kinase C inhibitor for use in a method of treating a solid tumor cancer in a patient in need thereof, the method comprising:
orally administering an effective amount of the protein kinase C inhibitor to the patient, wherein the patient has been determined to have a *GNAQ* or *GNA11* genetic tumor mutation, wherein the solid tumor cancer is selected from pancreatic adenocarcinoma, stomach adenocarcinoma, cervical carcinoma, and lung adenocarcinoma.

**9.** A protein kinase C inhibitor for use in a method of treating a non-uveal solid tumor cancer having a GNAQ or GNA11 genetic mutation in a patient in need thereof, wherein the non-uveal solid tumor cancer is selected from the group consisting of pancreatic cancer, stomach cancer, colorectal cancer, cervical cancer, lung adenocarcinoma, uterine cancer, bladder cancer, hepatocellular carcinoma, prostate cancer, breast cancer, head and neck cancer, and glioblastoma, the method comprising:

determining if the non-uveal solid tumor cancer has a *GNAQ* or *GNA11* genetic mutation;
determining if the patient does not have activating BRAF or NRAS mutations; and
orally administering to the patient without activating BRAF or NRAS mutations,
optionally wherein the patient does not have a BRAF V600 mutation, a pharmaceutically effective amount of the protein kinase C inhibitor,
optionally wherein the *GNAQ* or *GNA11* mutation is:

a. the substitution of glutamine in codon 209 (Q209) or the substitution of arginine in codon 183 (R183), optionally wherein the non-uveal solid tumor cancer is colorectal cancer, lung adenocarcinoma, or pancreatic cancer; or
b. other than a substitution of glutamine in codon 209 (Q209) and other than a substitution of arginine in codon 183 (R183), optionally wherein the non-uveal solid tumor cancer is uterine cancer, stomach cancer, cervical cancer, hepatocellular carcinoma, prostate cancer, breast cancer, head and neck cancer, or glioblastoma.

**10.** A protein kinase C inhibitor for use in a method of treating a patient having a non-uveal melanoma tumor or non-melanocytic tumor of the central nervous system, wherein the non-uveal melanoma tumor or non-melanocytic tumor of the central nervous system is pancreatic cancer, colorectal cancer, lung adenocarcinoma, stomach cancer, cervical cancer, uterine cancer, bladder cancer, hepatocellular carcinoma, prostate cancer, breast cancer, head and neck cancer, or glioblastoma, the method comprising:

determining if the non-uveal melanoma tumor or non-melanocytic tumor of the central nervous system has a *GNAQ* or *GNA11* genetic mutation that activates the PKC pathway; and
orally administering to the patient suffering from the non-uveal melanoma tumor or non-melanocytic tumor of the central nervous system with *GNAQ or GNA11* genetic mutation a pharmaceutically effective amount of the protein kinase C inhibitor,

optionally wherein determining comprises:

a. determining if the non-uveal melanoma tumor or non-melanocytic tumor of the central nervous system has a *GNAQ* Q209, *GNA11* Q209, *GNAQ* R183 or *GNA11* R183 genetic mutation, optionally wherein the non-uveal melanoma tumor or non-melanocytic tumor of the central nervous system is selected from the group consisting of a pancreatic cancer tumor, colorectal cancer tumor, and lung adenocarcinoma; or
b. determining if the non-uveal melanoma tumor or non-melanocytic tumor of the central nervous system has a mutation other than a *GNAQ* Q209, *GNA11* Q209, *GNAQ* R183 or *GNA11* R183 genetic mutation, optionally wherein the non-uveal melanoma tumor or non-melanocytic tumor of the central nervous system is uterine cancer, stomach cancer, cervical cancer, bladder cancer, hepatocellular carcinoma, prostate cancer, breast cancer, head and neck cancer, or glioblastoma.

**11.** A protein kinase C inhibitor for use in a method of treating a patient having a non-melanocytic tumor of the central nervous system, the method comprising:

determining if the non-melanocytic tumor of the central nervous system has a *GNAQ* or *GNA11* genetic mutation that activates the PKC pathway; and
orally administering to the patient suffering from the non-melanocytic tumor of the central nervous system with *GNAQ or GNA11* genetic mutation a pharmaceutically effective amount of the protein kinase C inhibitor,

optionally wherein determining comprises:

a. determining if the non-melanocytic tumor of the central nervous system has a *GNAQ* Q209, *GNA11* Q209, *GNAQ* R183, or *GNA11* R183 genetic mutation; or
b. determining if the non-melanocytic tumor of the central nervous system has a mutation other than a *GNAQ* Q209, *GNA11* Q209, *GNAQ* R183 or *GNA11* R183 genetic mutation, optionally wherein the non-melanocytic tumor of the central nervous system is glioblastoma.

**12.** A protein kinase C inhibitor for use in a method of treating a non-uveal solid tumor cancer in a patient in need thereof, wherein the patient is substantially non-responsive to treatment with an immune checkpoint inhibitor, the method comprising orally administering to the patient a pharmaceutically effective amount of the protein kinase C inhibitor, wherein the patient's non-uveal solid tumor cancer has been determined to have a *GNAQ* or *GNA11* genetic mutation, optionally wherein:

a. the non-uveal solid tumor cancer is selected from the group consisting of pancreatic cancer, stomach cancer, colorectal cancer, uterine cancer, cervical cancer, bladder cancer, hepatocellular carcinoma, head and neck cancer, prostate cancer, breast cancer, lung adenocarcinoma, and cutaneous melanoma, optionally wherein the patient's non-uveal solid tumor cancer has been determined to have a *GNAQ* Q209L or *GNA11* Q209L mutation; or
b. the patient's non-uveal solid tumor cancer has been determined to have a *GNAQ* Q209 or a *GNA11* Q209 mutation, optionally wherein the non-uveal solid tumor cancer is selected from the group consisting of pancreatic cancer, colorectal cancer, lung adenocarcinoma, and cutaneous melanoma; or
c. the patient's non-uveal solid tumor cancer has been determined to have a mutation other than a *GNAQ* Q209, *GNA11* Q209, *GNAQ* R183 or *GNA11* R183, optionally wherein the non-uveal solid tumor cancer is selected from the group consisting of uterine cancer, stomach cancer, bladder cancer, cervical cancer, breast cancer, head and neck cancer, hepatocellular carcinoma, and glioblastoma.

**13.** The protein kinase C inhibitor for use according to claim 12, wherein:

a. the patient is not responsive to a 3-month course of treatment with an immune checkpoint inhibitor; and/or
b. the immune check point inhibitor is selected from the group consisting of pembrolizumab, ipilimumab, nivolumab, and atezolizumab; and/or

c. the patient has a low tumor mutation burden; and/or
d. the patient has a low tumor load of mutations in one or more of BAP1, SF3B1, EIF1AX, TERT, BRAF, CDKN2A, NRAS and KRAS.

**14.** The protein kinase C inhibitor for use according to any one of claims 1-13, wherein the protein kinase C inhibitor is represented by Formula II:

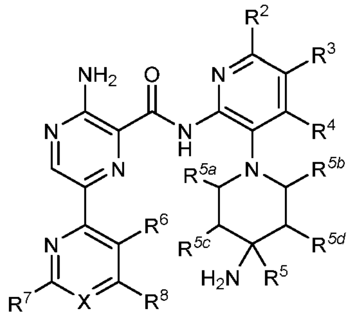

(II),

or a pharmaceutically acceptable salt thereof, wherein:

X is N or CR;
R, $R^2$, $R^3$, and $R^4$ are each independently selected from the group consisting of H, $^2H$, halogen, hydroxyl, $C_{1-3}$alkoxy and $C_{1-3}$alkyl; wherein $C_{1-3}$alkoxy may optionally be substituted by one, two, three or more halogens; and wherein $C_{1-3}$alkyl may optionally be substituted by one, two, three, or more substituents, each independently selected from the group consisting of hydroxyl, halogen and $C_{1-3}$alkoxy (optionally substituted by one or more halogens);
$R^5$ is selected from the group consisting of H, $^2H$, $-CH_3$, $-CH_2F$, $-CHF_2$, $-CF_3$, $-CH_2OH$, and $C_{2-3}$alkyl; wherein $C_{2-3}$alkyl may optionally be substituted by one, two, three, or more substituents, each independently selected from the group consisting of fluorine, hydroxyl, and $C_{1-3}$alkoxy (optionally substituted by one or more halogens);
$R^{5a}$ and $R^{5b}$ are each independently selected from the group consisting of H, $^2H$, and $C_{1-3}$alkyl; wherein $C_{1-3}$alkyl may optionally be substituted by one, two, three, or more substituents, each independently selected from the group consisting of fluorine, hydroxyl, and $C_{1-3}$alkoxy; or $R^{5a}$ and $R^{5b}$ are taken together to form a methylene or ethylene bridging group;
$R^{5c}$ and $R^{5d}$ are each independently selected from the group consisting of H, $^2H$, fluorine, hydroxyl, $C_{1-3}$alkoxy, and $C_{1-3}$alkyl; wherein $C_{1-3}$alkyl may optionally be substituted by one, two, three or more substituents, each independently selected from the group consisting of fluorine, hydroxyl and $C_{1-3}$alkoxy; or $R^{5c}$ and $R^{5d}$ taken together form a methylene, ethylene or $-CH_2$-O- bridging group;
$R^6$, $R^7$ and $R^8$ are each independently selected from the group consisting of H, $^2H$, halogen, $C_{1-3}$alkyl, $C_{1-3}$alkoxy, $C_{3-7}$cycloalkyl, and a 4-7 membered heterocyclyl having one, two, or three heteroatoms each independently selected from the group consisting of N, O, and S; wherein $C_{1-3}$alkoxy may optionally be substituted by one, two, three, or more halogens; and wherein $C_{1-3}$alkyl may optionally be substituted by one, two, three, or more substituents, each independently selected from the group consisting of hydroxyl, halogen, and $C_{1-3}$alkoxy (optionally substituted by one or more halogens); or
$R^6$ and $R^8$ form a partially unsaturated carbobicyclic or heterobicyclic ring with the heteroaryl ring to which they are attached, wherein the carbobicyclic or heterobicyclic ring may optionally be substituted by one, two, or three groups, each independently selected from the group consisting of $^2H$, halogen, $C_{1-3}$alkyl, $C_{1-3}$alkoxy, $C_{3-7}$cycloalkyl, and a 4-7 membered heterocyclyl having one, two, or three heteroatoms each independently selected from the group consisting of N, O, and S; and
wherein $C_{1-3}$alkyl and $C_{1-3}$alkoxy may optionally be substituted by one, two, three, or more halogens,

optionally wherein the protein kinase C inhibitor is:

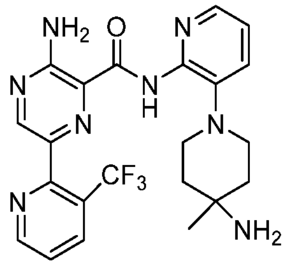,

or a pharmaceutically acceptable salt thereof.

**15.** A compound represented by:

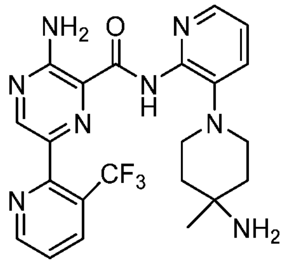,

or a pharmaceutically acceptable salt thereof,
for use in a method of treating a cancer in patient in need thereof, the method comprising:

identifying the patient as having a tumor with a *GNAQ* or *GNA11* mutation; orally administering a composition comprising the compound to the patient;
wherein the cancer is selected from the group consisting of pancreatic cancer tumor, stomach cancer tumor, colorectal cancer tumor, cervical cancer tumor, lung adenocarcinoma, cutaneous melanoma, colorectal cancer, stomach cancer, bladder cancer, hepatocellular carcinoma, prostate cancer, breast cancer, head and neck cancer, and glioblastoma.

**16.** The compound for use according to claim 15, wherein the cancer is cutaneous melanoma.

## Patentansprüche

**1.** Proteinkinase-C-Inhibitor zur Verwendung in einem Verfahren zur Behandlung eines Karzinoms mit einer genetischen *GNAQ*- oder *GNA11*-Mutation in einem Patienten, der dessen bedarf, wobei das Karzinom ausgewählt ist aus der Gruppe bestehend aus Adenokarzinom der Bauchspeicheldrüse, Adenokarzinom des Magens, Zervixkarzinom und Lungenadenokarzinom, wobei das Verfahren umfasst:

Bestimmen, ob das Karzinom eine genetische *GNAQ*- oder GNA11-Mutation aufweist; und
orales Verabreichen einer therapeutisch wirksamen Menge des Proteinkinase-C-Inhibitors an den Patienten, der das Karzinom mit der genetischen *GNAQ*- oder *GNA11*-Mutation aufweist.

**2.** Proteinkinase-C-Inhibitor zur Verwendung nach Anspruch 1, wobei die*GNAQ*- oder *GNA11*-Mutation eine Substitutions-, Insertions- und/oder Deletionsmutation und/oder eine Funktionszugewinn-Mutation ist.

**3.** Proteinkinase-C-Inhibitor zur Verwendung nach Anspruch 1 oder 2, wobei:

a. es sich bei der genetischen *GNAQ*- oder *GNA11*-Mutation um die Substitution von Glutamin in Codon 209 (Q209) handelt; und/oder
b. das Karzinom eine genetische *GNAQ*-Mutation, ausgewählt aus Q209P, Q209L, Q209H, Q209K und Q209Y, oder eine genetische *GNA11*-Mutation, ausgewählt aus Q209P, Q209L, Q209K und Q209H, aufweist; und/oder
c. es sich bei der genetischen *GNAQ*- oder *GNA11*-Mutation um Q209L handelt.

**4.** Proteinkinase-C-Inhibitor zur Verwendung nach Anspruch 1 oder 2, wobei:

a. es sich bei der genetischen *GNAQ*- oder *GNA11*-Mutation um die Substitution von Arginin in Codon 183 (R183) handelt, optional wobei es sich bei der genetischen *GNAQ*-Mutation um R183Q handelt; oder
b. die genetische *GNAQ*- oder *GNA11*-Mutation verschieden ist von einer Substitution des Glutamins in Codon 209 (Q209) und einer Substitution des Arginins in Codon 183 (R183), optional wobei die genetische *GNAQ*- oder *GNA11*-Mutation den PKC-Weg aktiviert.

**5.** Proteinkinase-C-Inhibitor zur Verwendung nach einem der Ansprüche 1-4, wobei:

a. das Bestimmen, ob das Karzinom eine genetische *GNAQ*- oder *GNA11*-Mutation aufweist, das Identifizieren der genetischen *GNAQ*- oder *GNA11*-Mutation in DNA, extrahiert aus einer Tumorprobe, oder in zirkulierender Tumor-DNA, umfasst; und/oder
b. das Bestimmen das Identifizieren der Anwesenheit eines Fusionsgens in DNA, extrahiert aus einer zirkulierenden Tumorzelle, umfasst; und/oder
c. der Patient eine geringe Tumor-Mutationslast von weniger als 17 mut/Mb aufweist, optional wobei der Patient eine geringe Tumor-Mutationslast von weniger als 10 mut/Mb aufweist; und/oder
d. der Patient eine geringe Tumor-Mutationslast von Mutationen über den Rest des Genoms hinweg aufweist; und/oder
e. der Patient keine aktivierenden BRAF- oder NRAS-Mutationen aufweist; und/oder
f. der Patient nicht auf Behandlung mit Immuncheckpoint-Inhibitoren angesprochen hat.

**6.** Proteinkinase-C-Inhibitor zur Verwendung nach einem der Ansprüche 1-5, wobei:

a. das Verabreichen das Verabreichen des Proteinkinase-C-Inhibitors als Monotherapie umfasst; oder
b. das Verabreichen ferner das Verabreichen wenigstens eines zusätzlichen therapeutischen Mittels umfasst; oder
c. das Verfahren ferner das Verabreichen einer therapeutisch wirksamen Menge eines therapeutischen Mittels umfasst, ausgewählt aus der Gruppe bestehend aus einem MEK-Inhibitor, einem mTOR-Inhibitor, einem $CDK_i$-Inhibitor, einem HDM2-p53-Inhibitor, einem Immuncheckpoint-Inhibitor und einer Kombination von zwei oder mehreren davon, optional wobei der MEK-Inhibitor aus der Gruppe bestehend aus Trametinib, Cobimetinib, Binimetinib und Selumetinib ausgewählt ist.

**7.** Proteinkinase-C-Inhibitor zur Verwendung nach einem der Ansprüche 1-6, wobei:

a. der Proteinkinase-C-Inhibitor Wirkstärke gegen eine oder mehrere der $\delta$-, $\varepsilon$-, $\eta$-, $\theta$-, $\alpha$-Proteinkinase-C-Isoformen aufweist; und/oder
b. der Proteinkinase-C-Inhibitor Wirkstärke gegen mehrere Proteinkinase-C-Isoformen aufweist; und/oder
c. der Proteinkinase-C-Inhibitor ein kleinmoleküliger Proteinkinase-C-Inhibitor ist; und/oder
d. der Proteinkinase-C-Inhibitor eine $IC_{50}$ in Bezug auf PKC $\theta/\alpha$- und/oder PKC $\delta$- und/oder PKC $\varepsilon$-Isoformen von weniger als 50 nM aufweist.

**8.** Proteinkinase-C-Inhibitor zur Verwendung in einem Verfahren zur Behandlung eines soliden Tumor-Krebses in einem Patienten, der dessen bedarf, wobei das Verfahren umfasst:
orales Verabreichen einer wirksamen Menge des Proteinkinase-C-Inhibitors an den Patienten, wobei bei dem Patienten festestellt wurde, dass er eine genetische *GNAQ*- oder *GNA11*-Tumormutation aufweist, wobei der solide Tumor-Krebs ausgewählt ist aus Adenokarzinom der Bauchspeicheldrüse, Adenokarzinom des Magens, Zervixkarzinom und Lungenadenokarzinom.

**9.** Proteinkinase-C-Inhibitor zur Verwendung in einem Verfahren zur Behandlung eines nicht-uvealen soliden Tumor-Krebses mit einer genetischen GNAQ- oder GNA11-Mutation in einem Patienten, der dessen bedarf, wobei der nicht-uveale solide Tumor-Krebs ausgewählt ist aus der Gruppe bestehend aus Bauchspeicheldrüsenkrebs, Magenkrebs, Kolorektalkrebs, Zervixkrebs, Lungenadenokarzinom, Uteruskrebs, Blasenkrebs, hepatozellulärem Karzinom, Prostatakrebs, Brustkrebs, Kopf/Hals-Krebs und Glioblastom, wobei das Verfahren umfasst:

Bestimmen, ob der nicht-uveale solide Tumor-Krebs eine genetische *GNAQ*- oder *GNA11*-Mutation aufweist;
Bestimmen, ob der Patient keine aktivierenden BRAF- oder NRAS-Mutationen aufweist; und
orales Verabreichen, an den Patienten, der keine aktivierenden BRAF- oder NRAS-Mutationen aufweist, optional

wobei der Patient keine BRAF-V600-Mutation aufweist, einer pharmazeutisch wirksamen Menge des Proteinkinase-C-Inhibitors, optional wobei die *GNAQ*- oder *GNA11*-Mutation:

a. die Substitution von Glutamin in Codon 209 (Q209) oder die Substitution von Arginin in Codon 183 (R183) ist, optional wobei es sich bei dem nicht-uvealen soliden Tumor-Krebs um Kolorektalkrebs, Lungenadenokarzinom oder Bauchspeicheldrüsenkrebs handelt; oder
b. verschieden ist von einer Substitution des Glutamins in Codon 209 (Q209) und verschieden ist von einer Substitution des Arginins in Codon 183 (R183), optional wobei es sich bei dem nicht-uvealen soliden Tumor-Krebs um Uteruskrebs, Magenkrebs, Zervixkrebs, hepatozelluläres Karzinom, Prostatakrebs, Brustkrebs, Kopf/Hals-Krebs oder Glioblastom handelt.

**10.** Proteinkinase-C-Inhibitor zur Verwendung in einem Verfahren zur Behandlung eines Patienten, der einen nicht-uvealen Melanom-Tumor oder nicht-melanozytären Tumor des Zentralnervensystems aufweist, wobei es sich bei dem nicht-uvealen Melanom-Tumor oder nicht-melanozytären Tumor des Zentralnervensystems um Bauchspeicheldrüsenkrebs, Kolorektalkrebs, Lungenadenokarzinom, Magenkrebs, Zervixkrebs, Uteruskrebs, Blasenkrebs, hepatozelluläres Karzinom, Prostatakrebs, Brustkrebs, Kopf/Hals-Krebs oder Glioblastom handelt, wobei das Verfahren umfasst:

Bestimmen, ob der nicht-uveale Melanom-Tumor oder nicht-melanozytäre Tumor des Zentralnervensystems eine genetische *GNAQ*- oder *GNA11*-Mutation, die den PKC-Weg aktiviert, aufweist; und
orales Verabreichen, an den Patienten, der an dem nicht-uvealen Melanom-Tumor oder nicht-melanozytären Tumor des Zentralnervensystems mit genetischer *GNAQ- oder GNA11*-Mutation leidet, einer pharmazeutisch wirksamen Menge des Proteinkinase-C-Inhibitors,
optional wobei das Bestimmen umfasst:

a. Bestimmen, ob der nicht-uveale Melanom-Tumor oder nicht-melanozytäre Tumor des Zentralnervensystems eine genetische *GNAQ* Q209-, *GNA11* Q209-, *GNAQ* R183- oder *GNA11* R183-Mutation aufweist, optional wobei der nicht-uveale Melanom-Tumor oder nicht-melanozytäre Tumor des Zentralnervensystems ausgewählt ist aus der Gruppe bestehend aus einem Krebstumor der Bauchspeicheldrüse, einem kolorektalen Krebstumor und Lungenadenokarzinom; oder
b. Bestimmen, ob der nicht-uveale Melanom-Tumor oder nicht-melanozytäre Tumor des Zentralnervensystems eine Mutation, verschieden von einer genetischen *GNAQ* Q209-, *GNA11* Q209-, *GNAQ* R183- oder *GNA11* R183-Mutation, aufweist, optional wobei der nicht-uveale Melanom-Tumor oder nicht-melanozytäre Tumor des Zentralnervensystems Uteruskrebs, Magenkrebs, Zervixkrebs, Blasenkrebs, hepatozelluläres Karzinom, Prostatakrebs, Brustkrebs, Kopf/Hals-Krebs oder Glioblastom ist.

**11.** Proteinkinase-C-Inhibitor zur Verwendung in einem Verfahren zur Behandlung eines Patienten, der einen nicht-melanozytären Tumor des Zentralnervensystems aufweist, wobei das Verfahren umfasst:

Bestimmen, ob der nicht-melanozytäre Tumor des Zentralnervensystems eine genetische *GNAQ*- oder *GNA11*-Mutation, die den PKC-Weg aktiviert, aufweist; und
orales Verabreichen, an den Patienten, der an dem nicht-melanozytären Tumor des Zentralnervensystems mit genetischer *GNAQ- oder GNA11*-Mutation leidet, einer pharmazeutisch wirksamen Menge des Proteinkinase-C-Inhibitors, optional wobei das Bestimmen umfasst:

a. Bestimmen, ob der nicht-melanozytäre Tumor des Zentralnervensystems eine genetische *GNAQ* Q209-, *GNA11* Q209-, *GNAQ* R183- oder *GNA11* R183-Mutation aufweist; oder
b. Bestimmen, ob der nicht-melanozytäre Tumor des Zentralnervensystems eine Mutation, verschieden von einer genetischen *GNAQ* Q209-, *GNA11* Q209-, *GNAQ* R183- oder *GNA11* R183-Mutation, aufweist, optional wobei der nicht-melanozytäre Tumor des Zentralnervensystems Glioblastom ist.

**12.** Proteinkinase-C-Inhibitor zur Verwendung in einem Verfahren zur Behandlung eines nicht-uvealen soliden Tumor-Krebses in einem Patienten, der dessen bedarf, wobei der Patient im Wesentlichen nicht auf Behandlung mit einem Immuncheckpoint-Inhibitor anspricht, wobei das Verfahren das orale Verabreichen, an den Patienten, einer pharmazeutisch wirksamen Menge des Proteinkinase-C-Inhibitors umfasst, wobei von dem nicht-uvealen soliden Tumor-Krebs des Patienten festgestellt wurde, eine genetische *GNAQ*- oder *GNA11*-Mutation aufzuweisen, optional wobei:

a. der nicht-uveale solide Tumor-Krebs ausgewählt ist aus der Gruppe bestehend aus Bauchspeicheldrüsenkrebs, Magenkrebs, Kolorektalkrebs, Uteruskrebs, Zervixkrebs, Blasenkrebs, hepatozellulärem Karzinom, Kopf/Hals-Krebs, Prostatakrebs, Brustkrebs, Lungenadenokarzinom und kutanem Melanom, optional wobei von dem nicht-uvealen soliden Tumor-Krebs des Patienten festgestellt wurde, eine *GNAQ* Q209L- oder *GNA11* Q209L-Mutation aufzuweisen; oder

b. von dem nicht-uvealen soliden Tumor-Krebs des Patienten festgestellt wurde, eine *GNAQ* Q209- oder eine *GNA11* Q209-Mutation aufzuweisen, optional wobei der nicht-uveale solide Tumor-Krebs ausgewählt ist aus der Gruppe bestehend aus Bauchspeicheldrüsenkrebs, Kolorektalkrebs, Lungenadenokarzinom und kutanem Melanom; oder

c. von dem nicht-uvealen soliden Tumor-Krebs des Patienten festgestellt wurde, eine Mutation, verschieden von *GNAQ* Q209, *GNA11* Q209, *GNAQ* R183 oder *GNA11* R183, aufzuweisen, optional wobei der nicht-uveale solide Tumor-Krebs ausgewählt ist aus der Gruppe bestehend aus Uteruskrebs, Magenkrebs, Blasenkrebs, Zervixkrebs, Brustkrebs, Kopf/Hals-Krebs, hepatozellulärem Karzinom und Glioblastom.

**13.** Proteinkinase-C-Inhibitor zur Verwendung nach Anspruch 12, wobei:

a. der Patient nicht auf einen 3-Monate-Behandlungszyklus mit einem Immuncheckpoint-Inhibitor anspricht; und/oder

b. der Immuncheckpoint-Inhibitor ausgewählt ist aus der Gruppe bestehend aus Pembrolizumab, Ipilimumab, Nivolumab und Atezolizumab; und/oder

c. der Patient eine geringe Tumor-Mutationslast aufweist; und/oder

d. der Patient eine geringe Tumor-Mutationenlast in einem oder mehreren von BAP1, SF3B1, EIF1AX, TERT, BRAF, CDKN2A, NRAS und KRAS aufweist.

**14.** Proteinkinase-C-Inhibitor zur Verwendung nach einem der Ansprüche 1-13, wobei der Proteinkinase-C-Inhibitor repräsentiert wird durch Formel II:

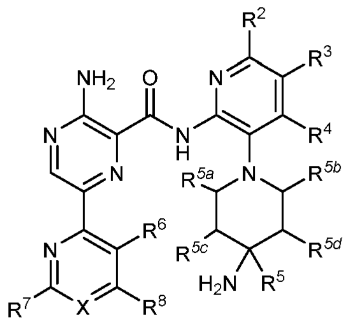

(II),

oder ein pharmazeutisch akzeptables Salz davon, wobei:

X für N oder CR steht;

R, $R^2$, $R^3$ und $R^4$ jeweils unabhängig ausgewählt sind aus der Gruppe bestehend aus H, $^2H$, Halogen, Hydroxyl, $C_{1-3}$-Alkoxy und $C_{1-3}$-Alkyl; wobei $C_{1-3}$-Alkoxy optional substituiert sein kann durch ein, zwei, drei oder mehr Halogene; und wobei $C_{1-3}$-Alkyl optional substituiert sein kann durch ein, zwei, drei oder mehr Substituenten, die jeweils unabhängig ausgewählt sind aus der Gruppe bestehend aus Hydroxyl, Halogen und $C_{1-3}$-Alkoxy (optional substituiert durch ein oder mehrere Halogene);

$R^5$ ausgewählt ist aus der Gruppe bestehend aus H, $^2H$, - $CH_3$, -$CH_2F$, -$CHF_2$, -$CF_3$, -$CH_2OH$ und $C_{2-3}$-Alkyl; wobei $C_{2-3}$-Alkyl optional substituiert sein kann durch ein, zwei, drei oder mehr Substituenten, die jeweils unabhängig ausgewählt sind aus der Gruppe bestehend aus Fluor, Hydroxyl und $C_{1-3}$-Alkoxy (optional substituiert durch ein oder mehrere Halogene);

$R^{5a}$ und $R^{5b}$ jeweils unabhängig ausgewählt sind aus der Gruppe bestehend aus H, $^2H$ und $C_{1-3}$-Alkyl; wobei $C_{1-3}$-Alkyl optional substituiert sein kann durch ein, zwei, drei oder mehr Substituenten, die jeweils unabhängig ausgewählt sind aus der Gruppe bestehend aus Fluor, Hydroxyl und $C_{1-3}$-Alkoxy; oder $R^{5a}$ und $R^{5b}$ zur Bildung einer Methylen- oder Ethylen-Verbrückungsgruppe zusammengenommen sind;

$R^{5c}$ und $R^{5d}$ jeweils unabhängig ausgewählt sind aus der Gruppe bestehend aus H, $^2H$, Fluor, Hydroxyl, $C_{1-3}$-Alkoxy und $C_{1-3}$-Alkyl; wobei $C_{1-3}$-Alkyl optional substituiert sein kann durch ein, zwei, drei oder mehr Substituenten, die jeweils unabhängig ausgewählt sind aus der Gruppe bestehend aus Fluor, Hydroxyl und $C_{1-3}$-Alkoxy; oder $R^{5c}$ und $R^{5d}$ zusammengenommen eine Methylen-, Ethylen- oder - $CH_2$-O- -Verbrückungs-

gruppe bilden;
$R^6$, $R^7$ und $R^8$ jeweils unabhängig ausgewählt sind aus der Gruppe bestehend aus H, $^2H$, Halogen, $C_{1-3}$-Alkyl, $C_{1-3}$-Alkoxy, $C_{3-7}$-Cycloalkyl und einem 4-7-gliedrigen Heterocyclyl mit einem, zwei oder drei Heteroatomen, die jeweils unabhängig ausgewählt sind aus der Gruppe bestehend aus N, O und S; wobei $C_{1-3}$-Alkoxy optional substituiert sein kann durch ein, zwei, drei oder mehr Halogene; und wobei $C_{1-3}$-Alkyl optional substituiert sein kann durch ein, zwei, drei oder mehr Substituenten, die jeweils unabhängig ausgewählt sind aus der Gruppe bestehend aus Hydroxyl, Halogen und $C_{1-3}$-Alkoxy (optional substituiert durch ein oder mehrere Halogene); oder
$R^6$ und $R^8$ einen partiell ungesättigten carbobizyklischen oder heterobizyklischen Ring mit dem Heteroarylring bilden, an dem sie angeheftet sind, wobei der carbobizyklische oder heterobizyklische Ring optional substituiert sein kann durch ein, zwei oder drei Gruppen, jeweils unabhängig ausgewählt aus der Gruppe bestehend aus $^2H$, Halogen, $C_{1-3}$-Alkyl, $C_{1-3}$-Alkoxy, $C_{3-7}$-Cycloalkyl und einem 4-7-gliedrigen Heterocyclyl mit einem, zwei oder drei Heteroatomen, die jeweils unabhängig ausgewählt sind aus der Gruppe bestehend aus N, O und S; und wobei $C_{1-3}$-Alkyl und $C_{1-3}$-Alkoxy optional substituiert sein können durch ein, zwei, drei oder mehr Halogene,
optional wobei es sich bei dem Proteinkinase-C-Inhibitor handelt um:

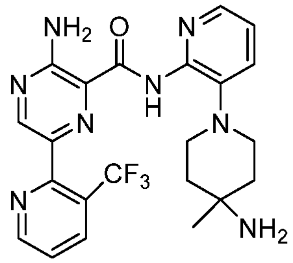
,

oder ein pharmazeutisch akzeptables Salz davon.

**15.** Verbindung, repräsentiert durch:

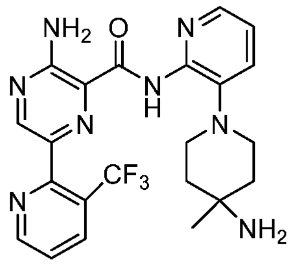
,

oder ein pharmazeutisch akzeptables Salz davon,
zur Verwendung in einem Verfahren zur Behandlung eines Krebses in einem Patienten, der dessen bedarf, wobei das Verfahren umfasst:

Identifizieren des Patienten als einen Tumor mit einer *GNAQ*- oder *GNA11*-Mutation aufweisend; orales Verabreichen einer die Verbindung umfassenden Zusammensetzung an den Patienten;
wobei der Krebs ausgewählt ist aus der Gruppe bestehend aus Krebstumor der Bauchspeicheldrüse, Magenkrebstumor, kolorektalem Krebstumor, Zervixkrebstumor, Lungenadenokarzinom, kutanem Melanom, Kolorektalkrebs, Magenkrebs, Blasenkrebs, hepatozellulärem Karzinom, Prostatakrebs, Brustkrebs, Kopf/Hals-Krebs und Glioblastom.

**16.** Verbindung zur Verwendung nach Anspruch 15, wobei es sich bei dem Krebs um kutanes Melanom handelt.

## Revendications

**1.** Inhibiteur de protéine kinase C pour une utilisation dans une méthode de traitement d'un carcinome présentant une mutation génétique de *GNAQ* ou de *GNA11* chez un patient en ayant besoin, le carcinome étant choisi dans le groupe constitué par un adénocarcinome du pancréas, un adénocarcinome de l'estomac, un carcinome du col de l'utérus et

un adénocarcinome du poumon, la méthode comprenant :

la détermination de la présence ou de l'absence d'une mutation génétique de *GNAQ* ou de *GNA11* dans le carcinome ; et
l'administration orale au patient atteint du carcinome présentant la mutation génétique de *GNAQ* ou de *GNA11*, d'une quantité thérapeutiquement efficace de l'inhibiteur de protéine kinase C.

**2.** Inhibiteur de protéine kinase C pour une utilisation selon la revendication 1, la mutation de *GNAQ* ou de *GNA11* étant une mutation par substitution, insertion et/ou délétion et/ou une mutation de gain de fonction.

**3.** Inhibiteur de protéine kinase C pour une utilisation selon la revendication 1 ou 2, **caractérisé en ce que** :

a. la mutation génétique de *GNAQ* ou de *GNA11* est la substitution de la glutamine au codon 209 (Q209) ; et/ou le carcinome présente une mutation génétique de *GNAQ* choisie parmi Q209P, Q209L, Q209H, Q209K et Q209Y, ou une mutation génétique de *GNA11* choisie parmi Q209P, Q209L, Q209K et Q209H ; et/ou
c. la mutation génétique de *GNAQ* ou de *GNA11* est Q209L.

**4.** Inhibiteur de protéine kinase C pour une utilisation selon la revendication 1 ou 2, **caractérisé en ce que** :

a. la mutation génétique de *GNAQ* ou de *GNA11* est la substitution de l'arginine au codon 183 (R183), facultativement, la mutation génétique de *GNAQ* étant R183Q ; ou
b. la mutation génétique de *GNAQ* ou de *GNA11* est autre qu'une substitution de la glutamine au codon 209 (Q209) et qu'une substitution de l'arginine au codon 183 (R183), facultativement, la mutation génétique de *GNAQ* ou de *GNA11* activant la voie PKC.

**5.** Inhibiteur de protéine kinase C pour une utilisation selon l'une quelconque des revendications 1 à 4, **caractérisé en ce que** :

a. la détermination de la présence ou de l'absence d'une mutation génétique de *GNAQ* ou de *GNA11* dans le carcinome comprend l'identification de la mutation génétique de *GNAQ* ou de *GNA11* dans de l'ADN extrait à partir d'un échantillon de tumeur ou dans de l'ADN tumoral circulant ; et/ou
b. la détermination comprend l'identification de la présence d'un gène de fusion dans de l'ADN extrait à partir d'une cellule tumorale circulante ; et/ou
c. le patient présente une faible charge mutationnelle tumorale, inférieure à 17 mut/Mb, facultativement, la charge mutationnelle tumorale étant inférieure à 10 mut/Mb ; et/ou
d. le patient présente une faible charge mutationnelle tumorale dans le reste du génome ; et/ou
e. le patient ne présente pas de mutations activatrices de BRAF ou de NRAS ; et/ou
f. le patient a été insensible au traitement par des inhibiteurs de points de contrôle immunitaire.

**6.** Inhibiteur de protéine kinase C pour une utilisation selon l'une quelconque des revendications 1 à 5, **caractérisé en ce que** :

a. l'administration comprend l'administration de l'inhibiteur de protéine kinase C en monothérapie ; ou
b. l'administration comprend en outre l'administration d'au moins un agent thérapeutique supplémentaire ; ou
c. la méthode comprend en outre l'administration d'une quantité thérapeutiquement efficace d'un agent thérapeutique choisi dans le groupe constitué par un inhibiteur de MEK, un inhibiteur de mTOR, un inhibiteur de $CDK_i$, un inhibiteur de HDM2-p53, un inhibiteur de point de contrôle immunitaire et une combinaison d'au moins deux de ces agents, facultativement, l'inhibiteur de MEK étant choisi dans le groupe constitué par le tramétinib, le cobimétinib, le binimétinib et le sélumétinib.

**7.** Inhibiteur de protéine kinase C pour une utilisation selon l'une quelconque des revendications 1 à 6, **caractérisé en ce que** :

a. l'inhibiteur de protéine kinase C est efficace contre une ou plusieurs des isoformes $\delta$, $\varepsilon$, $\eta$, $\theta$ et $\alpha$ de protéine kinase C ; et/ou
b. l'inhibiteur de protéine kinase C est efficace contre plusieurs isoformes de la protéine kinase C ; et/ou
c. l'inhibiteur de protéine kinase C est un inhibiteur de protéine kinase C à petite molécule ; et/ou
d. l'inhibiteur de protéine kinase C présente une $CI_{50}$ pour les isoformes PKC $\theta/\alpha$ et/ou PKC $\delta$ et/ou PKC $\varepsilon$

inférieure à 50 nM.

**8.** Inhibiteur de protéine kinase C pour une utilisation dans une méthode de traitement d'un cancer solide chez un patient en ayant besoin, la méthode comprenant :
l'administration orale d'une quantité efficace de l'inhibiteur de protéine kinase C au patient, la présence d'une mutation génétique tumorale de *GNAQ* ou de *GNA11* ayant été détectée chez le patient, le cancer solide étant choisi parmi un adénocarcinome du pancréas, un adénocarcinome de l'estomac, un adénocarcinome du col de l'utérus et un adénocarcinome du poumon.

**9.** Inhibiteur de protéine kinase C pour une utilisation dans une méthode de traitement d'un cancer solide non uvéal présentant une mutation génétique de GNAQ ou de GNA11 chez un patient en ayant besoin, le cancer solide non uvéal étant choisi dans le groupe constitué par un cancer du pancréas, un cancer de l'estomac, un cancer colorectal, un cancer du col de l'utérus, un adénocarcinome du poumon, un cancer de l'utérus, un cancer de la vessie, un cancer de la prostate, un cancer du sein, un cancer de la tête et du cou et un glioblastome, la méthode comprenant :

la détermination de la présence ou de l'absence d'une mutation génétique de *GNAQ* ou de *GNA11* dans le cancer solide non uvéal ;
la détermination de l'absence de mutations activatrices de BRAF ou de NRAS chez le patient ; et
l'administration orale au patient sans mutations activatrices de BRAF ou de NRAS, facultativement, le patient ne présentant pas la mutation de BRAF V600, d'une quantité pharmaceutiquement efficace de l'inhibiteur de protéine kinase C, facultativement, la mutation de *GNAQ* ou de *GNA11* étant :

a. la substitution de la glutamine au codon 209 (Q209) ou la substitution de l'arginine au codon 183 (R183), facultativement, le cancer solide non uvéal étant un cancer colorectal, un adénocarcinome du poumon ou un cancer du pancréas ; ou
b. autre qu'une substitution de la glutamine au codon 209 (Q209) et autre qu'une substitution de l'arginine au codon 183 (R183), facultativement, le cancer solide non uvéal étant un cancer de l'utérus, un cancer de l'estomac, un cancer du col de l'utérus, un carcinome hépatocellulaire, un cancer de la prostate, un cancer du sein, un cancer de la tête et du cou ou un glioblastome.

**10.** Inhibiteur de protéine kinase C pour une utilisation dans une méthode de traitement d'un patient atteint d'un mélanome non uvéal ou d'une tumeur non mélanocytaire du système nerveux central, le mélanome non uvéal ou la tumeur non mélanocytaire du système nerveux central étant un cancer du pancréas, un cancer colorectal, un adénocarcinome du poumon, un cancer de l'estomac, un cancer du col de l'utérus, un cancer de l'utérus, un cancer de la vessie, un carcinome hépatocellulaire, un cancer de la prostate, un cancer du sein, un cancer de la tête et du cou ou un glioblastome, la méthode comprenant :

la détermination de la présence ou de l'absence d'une mutation génétique de *GNAQ* ou de *GNA11* qui active la voie PKC dans le mélanome non uvéal ou la tumeur non mélanocytaire du système nerveux central ; et
l'administration orale au patient atteint d'un mélanome non uvéal ou d'une tumeur non mélanocytaire du système nerveux central présentant une mutation génétique de *GNAQ* ou de *GNA11,* d'une quantité pharmaceutiquement efficace de l'inhibiteur de protéine kinase C,
facultativement, la détermination comprenant :

a. la détermination de la présence ou de l'absence d'une mutation génétique de *GNAQ* Q209, *GNA11* Q209, *GNAQ* R183 ou de *GNA11* R183 dans le mélanome non uvéal ou la tumeur non mélanocytaire du système nerveux central, facultativement, le mélanome non uvéal ou la tumeur non mélanocytaire du système nerveux central étant choisi dans le groupe constitué d'une tumeur du pancréas, d'une tumeur colorectale et d'un adénocarcinome du poumon ; ou
b. la détermination de la présence ou de l'absence d'une mutation autre qu'une mutation génétique de *GNAQ* Q209, de *GNA11* Q209, de *GNAQ* R183 ou de *GNA11* R183 dans le mélanome non uvéal ou la tumeur non mélanocytaire du système nerveux central, facultativement, le mélanome non uvéal ou la tumeur non mélanocytaire du système nerveux central étant un cancer de l'utérus, un cancer de l'estomac, un cancer du col de l'utérus, un cancer de la vessie, un carcinome hépatocellulaire, un cancer de la prostate, un cancer du sein, un cancer de la tête et du cou ou un glioblastome.

**11.** Inhibiteur de protéine kinase C pour une utilisation dans une méthode de traitement d'un patient atteint d'une tumeur non mélanocytaire du système nerveux central, la méthode comprenant :

la détermination de la présence ou de l'absence d'une mutation génétique de *GNAQ* ou de *GNA11* qui active la voie PKC dans la tumeur non mélanocytaire du système nerveux central ; et
l'administration orale au patient atteint de la tumeur non mélanocytaire du système nerveux central présentant une mutation génétique de *GNAQ* ou de *GNA11,* d'une quantité pharmaceutiquement efficace de l'inhibiteur de protéine kinase C, facultativement, la détermination comprenant :

a. la détermination de la présence ou de l'absence d'une mutation génétique de *GNAQ* Q209, de *GNA11* Q209, de *GNAQ* R183 ou de *GNA11* R183 dans la tumeur non mélanocytaire du système nerveux central ; ou
b. la détermination de la présence ou de l'absence d'une mutation autre qu'une mutation génétique de *GNAQ* Q209, de *GNA11* Q209, de *GNAQ* R183 ou de *GNA11* R183 dans la tumeur non mélanocytaire du système nerveux central, facultativement, la tumeur non mélanocytaire du système nerveux central étant un glioblastome.

**12.** Inhibiteur de protéine kinase C pour une utilisation dans une méthode de traitement d'un cancer solide non uvéal chez un patient en ayant besoin, le patient étant pratiquement insensible au traitement par un inhibiteur de point de contrôle immunitaire, la méthode comprenant l'administration orale au patient d'une quantité pharmaceutiquement efficace de l'inhibiteur de protéine kinase C, la présence d'une mutation génétique de *GNAQ* ou de *GNA11* ayant été détectée dans le cancer solide non uvéal du patient, facultativement, **caractérisé en ce que** :

a. le cancer solide non uvéal est choisi dans le groupe constitué par un cancer du pancréas, un cancer de l'estomac, un cancer colorectal, un cancer de l'utérus, un cancer du col de l'utérus, un cancer de la vessie, un carcinome hépatocellulaire, un cancer de la tête et du cou, un cancer de la prostate, un cancer du sein, un adénocarcinome du poumon et un mélanome cutané, facultativement, la présence d'une mutation de *GNAQ* Q209L ou de *GNA11* Q209L ayant été détectée dans le cancer solide non uvéal du patient ; ou
b. la présence d'une mutation de *GNAQ* Q209 ou de *GNA11* Q209 a été détectée dans le cancer solide non uvéal du patient, facultativement, le cancer solide non uvéal étant choisi dans le groupe constitué par un cancer du pancréas, un cancer colorectal, un adénocarcinome du poumon et un mélanome cutané ; ou
c. la présence d'une mutation autre qu'une mutation de *GNAQ* Q209, de *GNA11* Q209, de *GNAQ* R183 ou de *GNA11* R183 a été détectée dans le cancer solide non uvéal du patient, facultativement, le cancer solide non uvéal étant choisi dans le groupe constitué par un cancer de l'utérus, un cancer de l'estomac, un cancer de la vessie, un cancer du col de l'utérus, un cancer du sein, un cancer de la tête et du cou, un carcinome hépatocellulaire et un glioblastome.

**13.** Inhibiteur de protéine kinase C pour une utilisation selon la revendication 12, **caractérisé en ce que** :

a. le patient est insensible à un traitement de 3 mois par un inhibiteur de point de contrôle immunitaire ; et/ou
b. l'inhibiteur de point de contrôle immunitaire est choisi dans le groupe constitué par le pembrolizumab, l'ipilimumab, le nivolumab et l'atézolizumab ; et/ou
c. le patient présente une faible charge mutationnelle tumorale ; et/ou
d. le patient présente une faible charge tumorale de mutations dans l'un ou plusieurs parmi BAP1, SF3B1, EIF1AX, TERT, BRAF, CDKN2A, NRAS et KRAS.

**14.** Inhibiteur de protéine kinase C pour une utilisation selon l'une quelconque des revendications 1 à 13, l'inhibiteur de protéine kinase C étant représenté par la formule II :

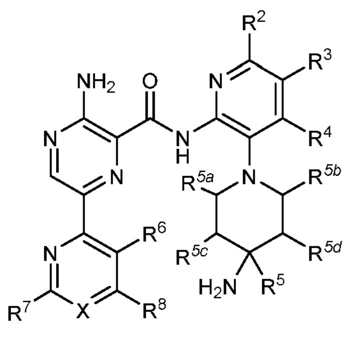

(II),

ou un sel pharmaceutiquement acceptable de celui-ci, **caractérisé en ce que** :

X est N ou CR ;

R, $R^2$, $R^3$ et $R^4$ sont chacun indépendamment choisis dans le groupe constitué par H, $^2H$, halogène, hydroxyle, alcoxy en $C_{1-3}$ et alkyle en $C_{1-3}$ ; alcoxy en $C_{1-3}$ pouvant facultativement être substitué par un, deux, trois ou plus de trois atomes d'halogène ; et alkyle en $C_{1-3}$ pouvant facultativement être substitué par un, deux, trois ou plus de trois substituants, chacun indépendamment choisi dans le groupe constitué par hydroxyle, halogène et alcoxy en $C_{1-3}$(facultativement substitué par un ou plusieurs atomes d'halogène) ;

$R^5$ est choisi dans le groupe constitué par H, $^2H$, $-CH_3$, $-CH_2F$, $-CHF_2$, $-CF_3$, $-CH_2OH$ et alkyle en $C_{2-3}$ ; alkyle en $C_{2-3}$ pouvant facultativement être substitué par un, deux, trois ou plus de trois substituants, chacun indépendamment choisi dans le groupe constitué par fluor, hydroxyle et alcoxy en $C_{1-3}$ (facultativement substitué par un ou plusieurs atomes d'halogène) ;

$R^{5a}$ et $R^{5b}$ sont chacun indépendamment choisis dans le groupe constitué par H, $^2H$ et alkyle en $C_{1-3}$ ; alkyle en $C_{1-3}$pouvant facultativement être substitué par un, deux, trois ou plus de trois substituants, chacun indépendamment choisi dans le groupe constitué par fluor, hydroxyle et alcoxy en $C_{1-3}$ ; ou $R^{5a}$ et $R^{5b}$ pris ensemble, forment un groupe pontant méthylène ou éthylène ;

$R^{5c}$ et $R^{5d}$ sont chacun indépendamment choisis dans le groupe constitué par H, $^2H$, fluor, hydroxyle, alcoxy en $C_{1-3}$, et alkyle en $C_{1-3}$ ; alkyle en $C_{1-3}$ pouvant facultativement être substitué par un, deux, trois ou plus de trois substituants, chacun indépendamment choisi dans le groupe constitué par fluor, hydroxyle et alcoxy en $C_{1-3}$ ; ou $R^{5c}$ et $R^{5d}$ pris ensemble, forment un groupe pontant méthylène, éthylène ou $-CH_2-O-$ ;

$R^6$, $R^7$ et $R^8$ sont chacun indépendamment choisis dans le groupe constitué par H, $^2H$, halogène, alkyle en $C_{1-3}$, alcoxy en $C_{1-3}$, cycloalkyle en $C_{3-7}$ et un hétérocyclyle de 4 à 7 chaînons comportant un, deux ou trois hétéroatomes chacun indépendamment choisis dans le groupe constitué par N, O et S ; alcoxy en $C_{1-3}$ pouvant facultativement être substitué par un, deux, trois ou plus de trois atomes d'halogène ; et alkyle en $C_{1-3}$ pouvant facultativement être substitué par un, deux, trois ou plus de trois substituants, chacun indépendamment choisi dans le groupe constitué par fluor, hydroxyle et alcoxy en $C_{1-3}$(facultativement substitué par un ou plusieurs atomes d'halogène) ; ou

$R^6$ et $R^8$ forment un cycle carbobicyclique ou hétérobicyclique partiellement insaturé avec le cycle hétéroaryle auquel ils sont liés, le cycle carbobicyclique ou hétérobicyclique pouvant être facultativement substitué par un, deux ou trois groupes, chacun indépendamment choisi dans le groupe constitué par $^2H$, halogène, alkyle en $C_{1-3}$, alcoxy en $C_{1-3}$, cycloalkyle en $C_{3-7}$, et un hétérocyclyle de 4 à 7 chaînons comportant un, deux ou trois hétéroatomes, chacun indépendamment choisi dans le groupe constitué par N, O et S ; et

alkyle en $C_{1-3}$ et alcoxy en $C_{1-3}$ peuvent être facultativement substitués par un, deux, trois ou plus de trois atomes d'halogène,

facultativement, l'inhibiteur de protéine kinase C étant :

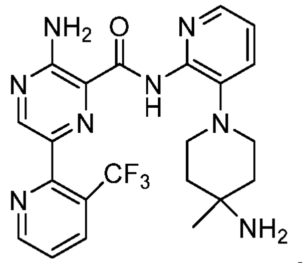

ou un sel pharmaceutiquement acceptable de celui-ci.

**15.** Composé représenté par :

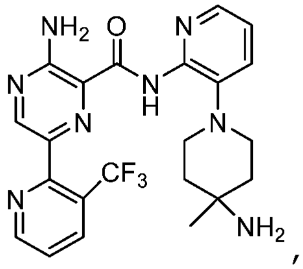

ou un sel pharmaceutiquement acceptable de celui-ci,
pour une utilisation dans une méthode de traitement d'un cancer chez un patient en ayant besoin, la méthode comprenant :

l'identification du patient comme présentant une tumeur comportant une mutation de *GNAQ* ou de *GNA11 ;*
l'administration orale d'une composition comprenant le composé au patient ;
le cancer étant choisi dans le groupe constitué par un cancer du pancréas, un cancer de l'estomac, un cancer colorectal, un cancer du col de l'utérus, un adénocarcinome du poumon, un mélanome cutané, un cancer colorectal, un cancer de l'estomac, un cancer de la vessie, un carcinome hépatocellulaire, un cancer de la prostate, un cancer du sein, un cancer de la tête et du cou et un glioblastome.

**16.** Composé pour une utilisation selon la revendication 15, le cancer étant un mélanome cutané.

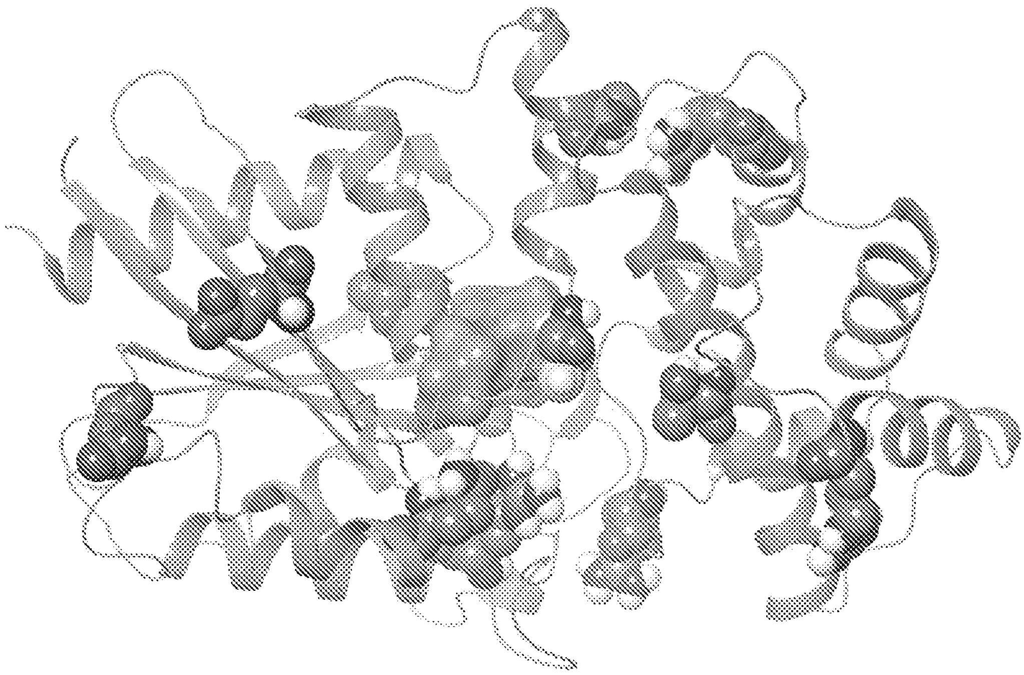

FIGURE 1

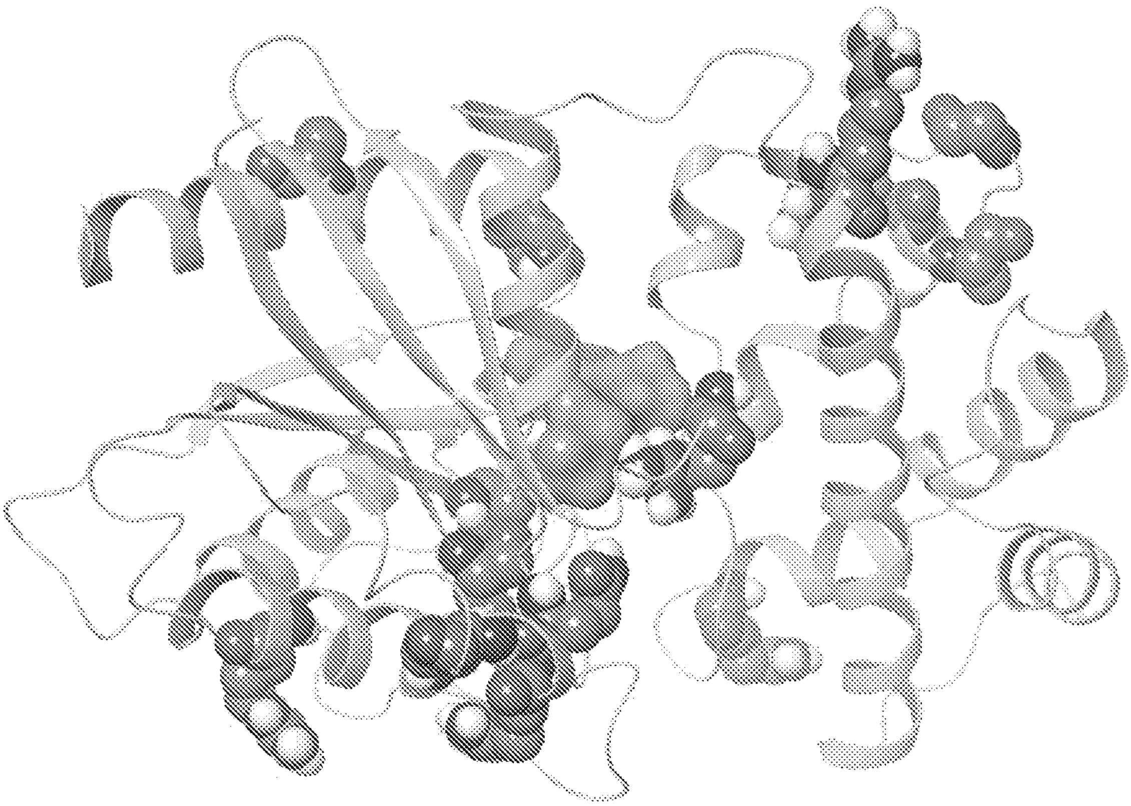

FIGURE 2

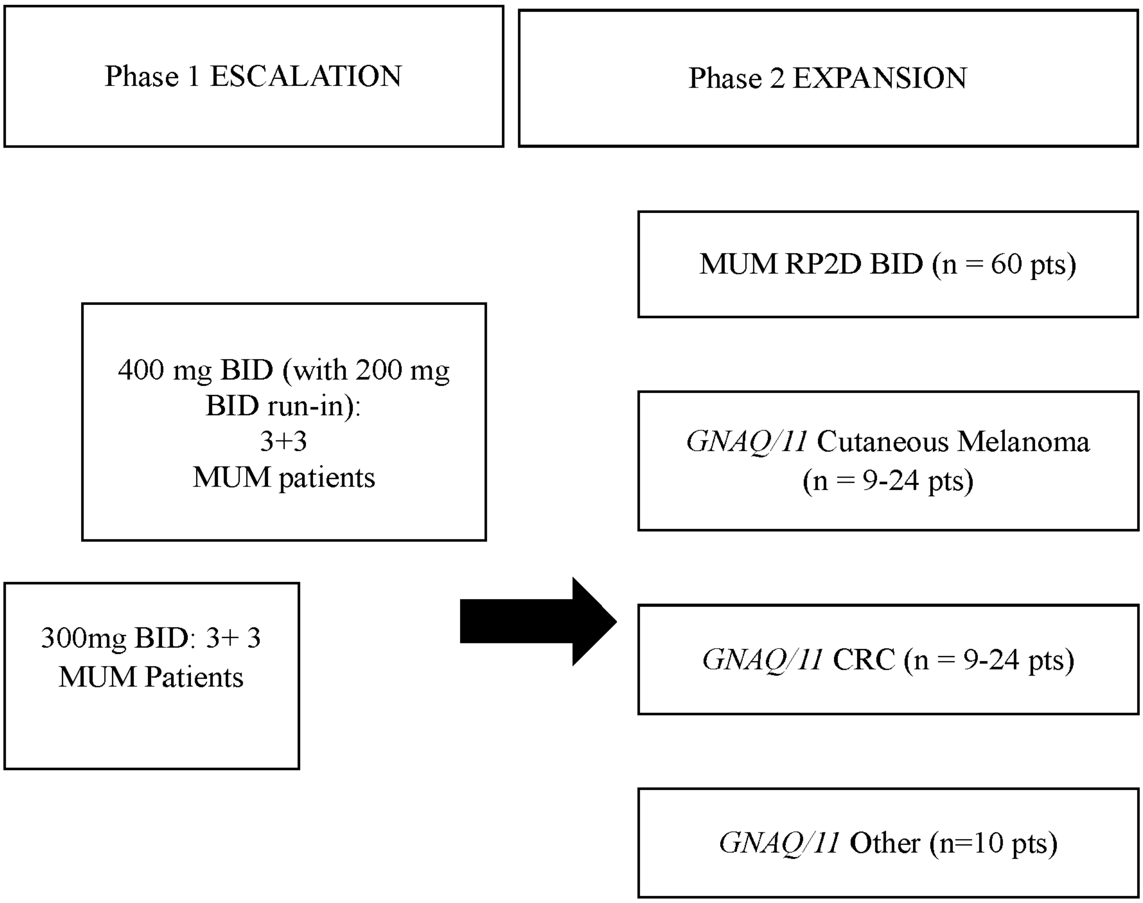
Phase 1 ESCALATION
Phase 2 EXPANSION
MUM RP2D BID (n = 60 pts)
400 mg BID (with 200 mg BID run-in): 3+3 MUM patients
*GNAQ/11* Cutaneous Melanoma (n = 9-24 pts)
300mg BID: 3+ 3 MUM Patients
*GNAQ/11* CRC (n = 9-24 pts)
*GNAQ/11* Other (n=10 pts)

**FIGURE 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62789177
- WO 2011130691 A
- WO 2016020864 A


### Non-patent literature cited in the description


- **KARLSSON A. et al.** antioxid. *Redox Signal.*, 2002, vol. 4, 49-60
- **HOWELL et al.** *Nat. Biotechnol.*, 1999, vol. 17, 87
- **LUO S** ; **MICHLER K** ; **JOHNSTON P et al.** A comparison of commonly used QT correction formulae: the effect of heart rate on the QTc of normal ECGs. *Journal of Electrocardiology.*, 2004, vol. 37, 81-90